# FASCICULE DE BREVET EUROPEEN

(11) **EP 2 252 886 B1**
(45) Date de publication et mention de la délivrance du brevet: **05.07.2017**
(21) Numéro de dépôt: 09727225.6
(22) Date de dépôt: 12.03.2009
(51) Int. Cl.: G01N 30/06, G01N 1/40, G01N 33/24

(54) **PROCEDE DE FONCTIONNALISATION EXTRACTIVE DE COMPOSES ORGANIQUES**
VERFAHREN ZUR EXTRAKTIVEN FUNKTIONALISIERUNG ORGANISCHER VERBINDUNGEN
METHOD FOR THE EXTRACTIVE FUNCTIONALIZING OF ORGANIC COMPOUNDS

(30) Priorité: 12.03.2008 FR 0851610
(43) Date de publication de la demande: 24.11.2010
(73) Titulaire: Centre National d'Etudes Spatiales (C.N.E.S.), 75001 Paris (FR)
(72) Inventeur: STERNBERG, Robert, F-94320 Thiais (FR); BUCH, Arnaud, F-75019 Paris (FR); CORREIA, Jean-Jacques, F-91120 Palaiseau (FR); CHAZALNOEL, Pascale, F-31280 Drémil-Lafage (FR)
(74) Mandataire: Novagraaf Technologies
(86) Numéro de dépôt international: PCT/FR2009/000263
(87) Numéro de publication internationale: WO 2009/122031

(56) Documents cités:
- EP-A- 1 136 141
- KR-A- 20040 059 205
- US-A- 5 637 209
- US-A- 5 716 525
- MEUNIER ET AL: "A laboratory pilot for in situ analysis of refractory organic matter in Martian soil by gas chromatography-mass spectrometry" 3 mai 2007 (2007-05-03), ADVANCES IN SPACE RESEARCH, PERGAMON, OXFORD, GB, PAGE(S) 337 - 344 , XP022059500 ISSN: 0273-1177 cité dans la demande abrégé figure 1 page 338, colonne de droite - page 339, colonne de gauche
- BUCH A ET AL: "Solvent extraction of organic molecules of exobiological interest for in situ analysis of the Martian soil" JOURNAL OF CHROMATOGRAPHY A 20030530 ELSEVIER NL, vol. 999, no. 1-2, 30 mai 2003 (2003-05-30), pages 165-174, XP002499634 cité dans la demande

## Description

### Domaine technique

La présente invention se rapporte à un procédé de fonctionnalisation extractive de composés organiques, par exemple de molécules organiques non-volatiles contenues dans un échantillon solide.

Le procédé de la présente invention consiste à traiter un échantillon afin d'extraire et de fonctionnaliser les molécules organiques qu'il contient, en vue par exemple de leur analyse. Il est utilisable par exemple pour l'analyse chimique *in situ* de composés organiques présents dans des sols terrestres ou extraterrestres. Le procédé de la présente invention peut être utilisé par exemple dans le domaine de l'exobiologie mais aussi dans d'autres domaines, tels que l'industrie alimentaire, l'industrie agronomique, l'industrie du parfum ou autres.

Dans la description ci-dessous, les références entre parenthèses **(ref x)** renvoient à la liste des références présentée après les exemples.

### Etat de la technique

L'analyse de sols et l'étude de leurs propriétés physiques, chimiques et biologiques a un intérêt dans de multiples domaines, par exemple dans le domaine de l'agriculture, de l'environnement ou de l'agroalimentaire. L'analyse de sols permet par exemple de mesurer la contamination ou la pollution de sols, d'évaluer l'impact de pratiques culturales, d'étudier le rôle des matières organiques, la fertilité ou le fonctionnement d'un sol, etc. En outre, ces informations peuvent permettre de préserver les ressources sols, de favoriser l'entretien ou la restauration de fonctions essentielles du sol, etc.

L'analyse de sols présente également un intérêt croissant dans le domaine de l'exobiologie. L'exobiologie est un domaine pluridisciplinaire qui inclut l'étude de l'origine, de l'évolution et de la distribution de la vie dans l'univers, y compris la Terre, la recherche de traces d'activités biologiques et l'étude de la chimie organique dans les milieux extraterrestres. Les molécules recherchées peuvent par exemple être des acides aminés, des acides carboxyliques, des bases nucléiques, etc.

L'analyse de molécules organiques présentes dans un échantillon solide, par exemple un échantillon de sol, nécessite des techniques qui permettent d'extraire, de séparer, d'identifier et éventuellement de quantifier les espèces moléculaires organiques et inorganiques présentes dans les échantillons prélevés. Ainsi, l'analyse de ces molécules nécessite généralement des techniques séparatives telles que la chromatographie en phase gazeuse (CPG) couplée ou non à la spectrométrie de masse (SM).

Cependant, l'analyse de molécules présentes dans un échantillon nécessite de traiter préalablement l'échantillon. Ce traitement consiste notamment à extraire les molécules de l'échantillon et à les transformer chimiquement et/ou physiquement, si nécessaire, afin de pouvoir les détecter et les analyser.

L'extraction des molécules nécessite des appareils et des conditions particulières. En effet, certaines molécules sont difficiles à extraire et sont fortement liées au solide. En général, l'extraction des molécules, est effectuée au moyen d'un solvant, cependant, l'extraction solide-liquide est difficile à mettre en oeuvre.

On peut citer par exemple l'extraction solide-liquide au moyen d'un appareil de soxhlet et d'un solvant approprié. Cependant, cette méthode nécessite un temps d'extraction assez long, d'environ 24 heures voire de plusieurs jours, avec un chauffage vigoureux du mélange solide-liquide, ce qui peut entraîner la dégradation de certaines molécules. De plus, elle nécessite un appareillage coûteux et encombrant. En outre, cette méthode est parfois insuffisante car certaines molécules sont difficiles à extraire du solide, par exemple certaines molécules de masses moléculaires élevées.

On peut également citer l'extraction en phase supercritique, par exemple en utilisant du CO₂ pressurisé, mais cette méthode est coûteuse. Par exemple, le document de Dadka A. et al., Journal of Hazardous Materials, 2002, 93 (3), page 307-320 **(ref 1)** décrit un procédé d'extraction de molécules organiques d'un sol en utilisant de l'eau en phase supercritique. Cependant, ceci nécessite un appareillage coûteux et encombrant, mais aussi de manipuler des fluides pressurisés. De plus, les substances à extraire doivent être solubles dans le fluide supercritique.

D'autres méthodes sont également utilisées, comme par exemple l'extraction en solvant par chauffage sous pression (ASE), nécessitant des fluides pressurisés avec de hautes températures et un appareillage coûteux et volumineux, ou encore l'extraction en solvant assistée par micro-ondes (MASE), nécessitant également un appareil coûteux et encombrant. En outre, certaines de ces méthodes ne sont pas adaptées à des applications spatiales, par exemple, l'extraction en solvant assistée par micro-ondes présente des risques d'interférence.

Ainsi, l'extraction de molécules fortement liées à la matrice qui les contient nécessite généralement des conditions dures qui entraînent souvent la dégradation de ces molécules. En outre, l'extraction est parfois difficile à mettre en oeuvre, et nécessite des dispositifs et appareillages encombrants. Elle ne peut donc pas être réalisée *in situ* sur le site de prélèvement de l'échantillon. De plus, les méthodes d'extraction existantes sont généralement utilisées pour des quantités relativement importantes d'échantillons et ne sont pas adaptées, notamment par leur encombrement, ni optimisées en termes de rendement pour des échantillons de faibles masses comme par exemple ceux utilisés pour l'analyse et la détection de molécules organiques dans des échantillons solides.

Par ailleurs, une fois les molécules extraites, elles doivent généralement être transformées de façon à être détectables et analysables par les instruments d'analyse couramment utilisés. La CPG nécessite notamment que les molécules à analyser soient volatilisées.

Ainsi, la pyrolyse est généralement utilisée comme procédé de transformation chimique et/ou physique de molécules organiques susceptibles d'être présentes dans un échantillon. Cette méthode conduit à la décomposition des molécules organiques en fragments généralement plus volatils.

A titre d'exemple, dans le cadre de l'exploration de la planète Mars, les deux sondes Viking ont permis d'effectuer des analyses chimiques in situ d'échantillons prélevés à la surface de Mars en utilisant à la fois un instrument de CPG classique (détecteur catharométrique) et un instrument de CPG-SM associés à des procédés de transformation chimique complexes destinés à détecter la matière organique et la présence d'une activité biologique par pyrolyse. Cependant, les sondes Viking n'ont pas permis de détecter des traces de matière organique à la surface de Mars. En effet, les instruments de CPG-SM de Viking ne pouvaient détecter que les composés gazeux ou vaporisables (par chauffage modéré) et par conséquent ne pouvaient pas accéder aux molécules considérées aujourd'hui comme d'intérêt exobiologique qui sont pour la plupart non volatiles.

Ainsi, de nombreux procédés utilisent la pyrolyse comme moyen de traitement en vue de l'analyse de composés organiques. A titre d'exemple, le document Mowry Curtis et al., Proceeding of SPIE, 2001, Vol. 4575, pages 83-90 **(ref 2)** décrit un procédé de détection rapide et d'identification de bactéries qui comprend une étape de prélèvement d'un échantillon, une étape de pyrolyse et une étape d'analyse par chromatographie en phase gazeuse.

Ainsi, la pyrolyse est une transformation chimique qui permet en effet l'obtention de composés (fragments) volatils. Cependant, cette transformation engendre la dégradation des molécules et conduit à la dénaturation de certaines propriétés physiques et chimiques des molécules cibles comme par exemple la chiralité ou l'asymétrie. Par conséquent elle pose des problèmes pour l'identification des molécules initiales à partir de leurs nombreux fragments de pyrolyse.

Par ailleurs, les procédés de traitement d'échantillon en vue de l'analyse des molécules qu'il contient sont généralement difficiles à mettre en oeuvre, comprenant généralement de nombreuses étapes et utilisant plusieurs appareillages et réacteurs, généralement encombrants et difficilement transportables sur un site de mesure. Des procédés de traitement et d'analyse d'échantillon sont par exemple décrits dans les documents Buch et al., Planetary and Space Science, Special Astrobiology Issue, 54, 15, (2006), 1592-1599 **(ref 3)** et Buch et al., Journal of Chromatography A. 999, (2003), 165-174 **(ref 4).**

Il existe donc un réel besoin de disposer d'un procédé d'extraction et de fonctionnalisation de molécules organiques contenues, adsorbées ou absorbées dans un échantillon solide, palliant ces défauts, inconvénients et obstacles de l'art antérieur. En outre, il existe un réel besoin de disposer d'un procédé de fonctionnalisation extractive compatible avec les contraintes d'encombrement et les contraintes spatiales, permettant de traiter et préparer *in situ* les échantillons prélevés de façon quantitative, sans dénaturation des composés et en un minimum d'étapes. En outre, il existe un réel besoin de disposer d'un procédé simple, rapide, avec de bons rendements et permettant d'optimiser la sensibilité, la qualité et l'interprétation des analyses.

### Description de l'invention

La présente invention a précisément pour but de répondre à ces besoins et inconvénients de l'art antérieur en fournissant un procédé d'extraction, de fonctionnalisation et de volatilisation de molécules organiques non-volatiles présentes dans un milieu solide comprenant les étapes suivantes :
(a) prélever un échantillon solide de sol ;
(b) mettre l'échantillon sous une pression P_{d} de 0,1 à 200 kPa, ;
(c) élever en moins de 30 secondes la température de l'échantillon de la température de l'environnement à une température T_{d} allant de 250°C à 600°C ;
(d) maintenir l'échantillon à la température T_{d} pendant une durée D_{d} inférieure à 15 minutes ;
(e) diminuer en moins de 30 secondes la température de l'échantillon de la température T_{d} à la température T_{f} allant de 50°C à 100°C ;
(f) ajouter à l'échantillon au moins un agent de fonctionnalisation et au moins un solvant organique, et maintenir l'échantillon à la température T_{f} pendant une durée D_{f} inférieure à 1 heure ; et
(g) chauffer l'échantillon à une température Tᵥ allant de 140°C à 300°C pendant une durée Dᵥ inférieure à 1 heure ce qui provoque une volatilisation des molécules organiques fonctionnalisées extraites de l'échantillon de sol.

On entend par « échantillon » au sens de la présente invention tout échantillon solide, susceptible de contenir des molécules organiques. Il peut s'agir par exemple d'échantillons de sol prélevé en surface ou en profondeur, provenant par exemple d'un grattage ou d'une carotte de sol. Il peut s'agir par exemple de différents types de sols, par exemple roche, gravas, pierres, sable, poussière, etc. d'origine terrestre ou extraterrestre. Il peut provenir par exemple d'une autre planète que la Terre, d'un satellite naturel ou d'une météorite. Il peut s'agir par exemple d'un sol provenant de Mars, la Lune, Titan, etc.

Au sens de la présente invention, l'échantillon peut comporter des molécules organiques, qui peuvent être volatiles ou non-volatiles, présentes ou non à l'état de traces. Il peut s'agir de molécules chimiques ou de molécules biologiques, comme par exemple des acides carboxyliques, des acides aminés, des peptides, des protéines, des sucres, des acides gras, des lipides, des bases puriques, des amines, des hydrocarbures aromatiques polycycliques (HAP), des acides et bases nucléiques, de l'ADN ou de fragments de ces molécules. On entend par « molécules organiques non-volatiles » au sens de la présente invention toute molécule organique qui ne passe pas à l'état gazeux par chauffage sans dégradation, c'est-à-dire dont la volatilisation entraîne la dégradation. Il peut par exemple s'agir de molécules organiques de la liste précitée.

On entend par « température de l'environnement » au sens de la présente invention la température du milieu comprenant l'échantillon.

Le procédé de la présente invention permet le traitement d'un échantillon et notamment :
- l'extraction de molécules organiques présentes dans l'échantillon,
- la fonctionnalisation des molécules organiques extraites de l'échantillon
- la volatilisation des molécules organiques fonctionnalisées.
En d'autres termes, le procédé de l'invention permet de détacher des molécules organiques, et notamment des molécules organiques non-volatiles, contenues dans un échantillon sans les détruire, de les fonctionnaliser afin de les rendre plus volatiles et de les volatiliser par exemple en vue de leur analyse.

Le procédé de la présente invention présente l'avantage de permettre de traiter (c'est-à-dire d'extraire, de fonctionnaliser et de volatiliser) les molécules organiques de l'échantillon, qualitativement et quantitativement.

Par « qualitativement », on entend au sens de la présente invention de façon à préserver la nature et la qualité des échantillons, c'est-à-dire de façon à limiter voir éviter la dégradation des molécules organiques lors des étapes d'extraction, de fonctionnalisation et de volatilisation, notamment lors de l'étape d'extraction. Par « quantitativement », on entend au sens de la présente invention de façon à traiter (c'est-à-dire extraire, fonctionnaliser et volatiliser) le plus possible de molécules organiques présentes dans l'échantillon. Le procédé de la présente invention permet avantageusement d'atteindre un bon rendement de récupération des molécules organiques, notamment supérieur à 80%, et donc d'obtenir une quantité suffisante de molécules organiques pour les analyser.

Selon un mode particulier de réalisation de l'invention, au moins une des étapes (c) à (g) peut être réalisée sous ultrasons ou sous micro-ondes.

Les étapes d'extraction, de fonctionnalisation (f) et de volatilisation (g) du procédé de l'invention sont décrites ci-dessous.

### Extraction de molécules organiques :

On entend par « extraction » au sens de la présente invention la séparation des molécules organiques de la matrice solide qui les contient. Par exemple, dans le cas d'un échantillon solide, les molécules organiques absorbées ou adsorbées à sa surface peuvent être fortement liées au solide et peuvent être très difficiles à extraire, d'où la nécessité de recourir à des méthodes d'extraction efficaces.

Selon le procédé de l'invention, l'extraction est réalisée par thermo-désorption. Ainsi, l'étape (d) du procédé de la présente invention consiste à maintenir l'échantillon à une température T_{d} pendant une durée D_{d} afin de désorber par thermo-désorption les molécules organiques contenues dans l'échantillon solide.

On entend par « thermo-désorption » au sens de la présente invention une méthode permettant de désorber des molécules organiques absorbées ou adsorbés sur un support solide, par chauffage à une température de désorption T_{d} et à une pression P_{d} pendant une durée D_{d}.

Selon l'invention, la température T_{d} est élevée, c'est à dire qu'elle peut aller de 250°C à 600°C. Par exemple, la température T_{d} peut être de 300 à 550 °C, par exemple de 300 à 500 °C. Avantageusement, au cours des étapes (c), (d) et (e), la température T_{d} peut être de 500°C +/-10°C, par exemple de 500°C +/- 5°C.

Selon l'invention, la pression P_{d} est de 0,1 à 200 kPa. La pression P_{d} peut être choisie de façon à faciliter la désorption, par exemple, l'extraction par thermo-désorption peut être effectuée sous pression réduite. Par exemple, la pression P_{d}, peut aller de 0,1 à 100 kPa, par exemple de 10 à 100 kPa. Des moyens utilisables d'application d'une pression sont décris ci-dessous.

Selon l'invention, au cours de l'étape (d), la durée D_{d} est inférieure à 15 minutes. Avantageusement, au cours de l'étape (d), la température T_{d} est maintenue pendant une durée D_{d} inférieure à 5 minutes. Selon un autre mode de réalisation de l'invention, la durée D_{d} peut être de 5 minutes +/- 1 minute.

Selon l'invention, l'étape (d) peut éventuellement être réalisée sous ultrasons ou sous micro-ondes afin de faciliter la désorption des molécules organiques.

Selon l'invention, au cours de l'étape (c), l'élévation de température est rapide, c'est-à-dire qu'elle s'effectue en moins de 30 secondes, par exemple, en moins de 15 secondes. Avantageusement, l'élévation de température peut s'effectuer en 15 secondes +/- 10 secondes. Ceci permet de contrôler avec précision le temps pendant lequel l'échantillon est maintenu à température élevée. En effet, au-delà d'un certain temps à température élevée, les molécules peuvent être dégradées. Ainsi, une élévation de température trop lente peut engendrer la dégradation des molécules organiques.

Selon l'invention, au cours de l'étape (e), la diminution de température est rapide, c'est-à-dire qu'elle s'effectue en moins de 30 secondes, par exemple, en moins de 15 secondes. Avantageusement, la diminution de température peut s'effectuer en 15 secondes +/- 10 secondes. Comme décrit précédemment, ceci permet de limiter les risques de dégradation des molécules organiques.

Ainsi, l'étape d'extraction par thermo-désorption selon le procédé de l'invention présente l'avantage d'être rapide et de ne nécessiter que quelques minutes. En effet, l'extraction, peut être réalisée en moins de 20 minutes, par exemple en moins de 15 minutes, par exemple en moins de 10 minutes.

De plus, le procédé de l'invention présente l'avantage d'extraire les molécules organiques sans les casser compte tenu des temps de chauffage appliqués très courts et des variations importantes de températures en des temps très cours.

En outre, l'étape d'extraction selon le procédé de l'invention permet de consommer moins d'énergie compte tenu des temps de chauffage très courts contrairement aux méthodes d'extraction généralement utilisées.

En outre, l'étape d'extraction par thermo-désorption selon le procédé de l'invention présente l'avantage d'éviter l'utilisation de solvants, contrairement aux méthodes d'extraction généralement utilisées. En effet, les solvants sont généralement dangereux, néfastes pour l'environnement et/ou coûteux. De plus, cette méthode permet d'éviter l'étape supplémentaire d'élimination du solvant d'extraction, ce qui présente plusieurs avantages :
- l'extraction selon le procédé de l'invention est réalisable en une seule étape, donc plus facile et plus rapide à mettre en oeuvre ;
- l'extraction selon le procédé de l'invention permet d'éviter les pertes en molécules organiques extraites qui peuvent parfois être en partie entraînées avec le solvant d'extraction lors de son élimination.

En outre, l'extraction par thermo-désorption selon le procédé de l'invention présente l'avantage d'être adaptée à de faibles masses de molécules organiques à extraire. En effet, les méthodes d'extraction existantes sont généralement utilisées pour des quantités relativement importantes d'échantillons et ne sont pas adaptées ni optimisées pour des échantillons présentant des molécules organiques à extraire en faibles quantités ou à l'état de traces.

L'extraction par thermo-désorption permet de détacher les molécules organiques de la matrice solide de l'échantillon, que ces molécules soient volatiles ou non-volatiles. En outre, cette méthode peut permettre de séparer les molécules organiques volatiles des molécules organiques non-volatiles. En effet, les molécules organiques volatiles se retrouvent à l'état gazeux suite à la thermo-désorption et peuvent être balayées dans un autre lieu, tandis que les molécules organiques non-volatiles restent dans l'enceinte avec le solide.

La facilité de mise en oeuvre de l'étape d'extraction par thermo-désorption selon le procédé de l'invention permet en outre de réaliser l'étape suivante de fonctionnalisation des molécules organiques extraites directement sur l'échantillon solide en un même lieu.

### Fonctionnalisation de molécules organiques extraites :

On entend par « fonctionnalisation » au sens de la présente invention la transformation chimique de molécules organiques en introduisant au moins un groupe fonctionnel afin de modifier leurs propriétés physico-chimiques comme par exemple leur volatilité.

Ainsi, le procédé de l'invention permet de fonctionnaliser les molécules organiques extraites de l'échantillon solide, afin de les rendre plus volatiles.

Ainsi, l'étape de fonctionnalisation (f) consiste à ajouter à l'échantillon au moins un agent de fonctionnalisation et au moins un solvant, et à maintenir l'échantillon à une température T_{f} pendant une durée D_{f} afin de faire réagir les molécules organiques extraites avec au moins un agent de fonctionnalisation pour obtenir des molécules organiques fonctionnalisées.

Selon l'invention, la température T_{f} est de 50°C à 100°C. Par exemple, la température T_{f}, peut être de 60 à 90°C. Avantageusement, au cours des étapes (e) et (f), la température T_{f} peut être de 75°C +/- 10°C, par exemple de 75°C +/- 5°C, par exemple de 75°C +/- 1°C.

Selon l'invention, la durée D_{f} est inférieure à 1 heure. Avantageusement, au cours de l'étape (f), l'agitation à la température T_{f} peut être maintenue pendant une durée D_{f} de 5 à 40 minutes, par exemple de 20 à 40 minutes. Selon un autre mode de réalisation de l'invention, la durée D_{f} peut être de 10 à 30 minutes.

On entend par « agent de fonctionnalisation » au sens de la présente invention un réactif pouvant se substituer à des groupements ou hydrogènes labiles des molécules organiques pour donner des molécules fonctionnalisées. Ces molécules fonctionnalisées sont généralement plus volatiles. En effet, la fonctionnalisation peut diminuer la polarité des molécules organiques et rendre moins fortes les liaisons hydrogène présentes au sein des molécules permettant ainsi leur volatilisation à de plus faibles températures, par exemple de l'ordre de 200°C.

Dans le cadre de la présente invention, parmi les agents de fonctionnalisation utilisables, on peut distinguer les agents de fonctionnalisation permettant ou non la mesure chromatographique de la chiralité des molécules organiques fonctionnalisées. Cette mesure consiste à détecter et à déterminer qualitativement et quantitativement la présence éventuelle de molécules énantiomères dans l'échantillon. En effet, certains agents de fonctionnalisation peuvent par exemple comprendre un centre chirale, ce qui permet après réaction avec une molécule organique de détecter d'éventuels isomères de la molécule organique fonctionnalisée et donc d'identifier si la molécule organique initiale est chirale ou non.

Selon un mode particulier de réalisation de l'invention, au cours de l'étape (f), l'agent de fonctionnalisation peut être choisi dans le groupe comprenant le *N*-méthyl-*N*-[tert-butyldiméthyl-silyl]trifluoroacétamide (MTBSTFA), le *N,N*-diméthylformamide diméthylacétal (DMF-DMA), l'hydroxyde de tétraméthylammonium (TMAH), l'anhydride trifluoroacétique (TFAA), l'anhydride heptafluorobutyrique (HFBA), le 2,2,2-trifluoroéthanol (TFE), le 2,2,3,3,4,4,4-heptafluoro-1-butanol (HFB), le chloroformate de méthyle (MCF), le chloroformate d'éthyle (ECF) ou un mélange de ceux-ci.

Le tableau 1 suivant présente, pour différents agents de fonctionnalisation, les actions ou substitutions possibles avec les groupements des molécules organiques à fonctionnaliser, ainsi que les propriétés ou non de mesurer la chiralité des molécules organiques.

**Tableau 1**

| **Agent de dérivatisation** | **Action et Propriétés** |
|---|---|
| MTBSTFA | Tous H labile - pas de chiralité |
| TMAH | Tous composés organiques - pas de chiralité |
| DMF-DMA | Acide aminé - chiralité |
| MCF | H labile - chiralité |
| ECF | H labile - chiralité |
| TFAA + TFE | H labile - chiralité |
| HFBA + HFB | H labile - chiralité |

On peut noter que le MTBSTFA est un agent de fonctionnalisation agressif pouvant avoir des conséquences sur l'enceinte dans laquelle est réalisée la fonctionnalisation d'où l'intérêt d'utiliser une enceinte dont les parois internes sont constituées ou recouvertes d'un matériau inattaquable. Des matériaux inattaquables sont décris ci-dessous.

Le solvant peut être tout solvant connu de l'homme du métier permettant la réaction de fonctionnalisation des molécules organiques par l'agent de fonctionnalisation. Selon un mode particulier de réalisation de l'invention, au cours de l'étape (f), le solvant peut être choisi dans le groupe comprenant l'eau, les alcanes en C₁ à C₈, linéaires, ramifiés ou cycliques, les alcools R^{S}-OH où R^{S} est un radical alkyle en C₁ à C₈, par exemple l'isopropanol, les cétones R^{S1}-C(=O)-R^{S2}, les éthers R^{S1}-O-R^{S2}, où R^{S1} et R^{S2} sont indépendamment des radicaux alkyles en C₁ à C₈, formant éventuellement un cycle, le tétrahydrofurane (THF), le dioxane, l'acétonitrile, le diméthylformamide (DMF), le diéthylformamide, le diméthylsulfoxyde, le toluène, l'acide acétique, l'acide formique, le dichlorométhane, le chloroforme, le dichloroéthane, l'acétate d'éthyle, ou un mélange de ceux-ci. On entend par « alkyle » au sens de la présente invention, un radical carboné linéaire, ramifié ou cyclique, saturé ou insaturé, éventuellement substitué, comprenant 1 à 8 atomes de carbone, par exemple 1 à 6 atomes de carbone. On peut noter à titre d'exemple que le DMF et l'eau permettent la solubilisation et l'extraction de molécules organiques comme les acides aminés tandis que l'isopropanol permet la solubilisation et l'extraction d'autres molécules organiques cibles.

Selon l'invention, l'étape (f) peut être réalisée à une pression P_{f} allant de 0,1 à 200 kPa, par exemple de 0,1 à 100 kPa, par exemple de 10 à 100 kPa. Des moyens permettant d'appliquer une telle pression sont décris ci-dessous.

Eventuellement, selon l'invention, l'étape (f) peut être réalisée sous agitation. Par exemple, elle peut être réalisée sous ultrasons ou sous micro-ondes afin de favoriser la réaction de fonctionnalisation des molécules organiques.

L'étape de fonctionnalisation (f) est précédée d'une étape (e) permettant d'atteindre la température de fonctionnalisation T_{f}. Selon un mode de réalisation particulier de l'invention, le procédé peut comprendre en outre, après l'étape (e) et avant l'étape (f), une étape (e') de stabilisation de la température qui consiste à maintenir la température T_{f} pendant une durée inférieure à 10 minutes, par exemple une durée de 2 à 5 minutes, par exemple une durée de 5 minutes +/- 1 minute. Cette étape permet de laisser l'échantillon atteindre la température T_{f} avant l'ajout de l'agent de fonctionnalisation.

### Volatilisation de molécules organiques fonctionnalisées :

On entend par « volatilisation » au sens de la présente invention le passage des molécules organiques à l'état gazeux.

Ainsi, une fois les molécules organiques fonctionnalisées, elles peuvent être volatilisées selon le procédé de l'invention notamment en vue de leur analyse. Ainsi, l'étape de volatilisation (g) consiste à chauffer l'échantillon à une température Tᵥ pendant une durée Dᵥ ce qui provoque une volatilisation des molécules organiques fonctionnalisés.

Selon l'invention, la température Tᵥ est de 140°C à 300°C. Par exemple, la température Tᵥ peut être de 180 à 200°C. Avantageusement, au cours de l'étape (g), la température Tᵥ peut être de 200°C +/- 20°C, par exemple de 200°C +/- 10°C.

Selon l'invention, la durée Dᵥ est inférieure à 1 heure. Par exemple, au cours de l'étape (g), la température Tᵥ peut être maintenue pendant une durée Dᵥ de 10 à 30 minutes.

Selon l'invention, la volatilisation des molécules organiques fonctionnalisées peut être effectuée à une pression Pᵥ allant de 0,1 à 200 kPa. Par exemple, au cours de l'étape (g), la pression Pᵥ peut être de 0,1 à 100 kPa, par exemple de 10 à 100 kPa. Des moyens permettant d'appliquer une telle pression sont décris ci-dessous.

L'étape de volatilisation (g) peut comprendre en outre une étape d'élévation de température à la température Tᵥ. Avantageusement, au cours de l'étape (g), l'élévation de température à la température Tᵥ peut être rapide, c'est-à-dire que la température Tᵥ peut être atteinte en moins de 30 secondes, par exemple, en moins de 15 secondes. Selon un autre mode de réalisation de l'invention, l'élévation de la température de l'échantillon à la température Tᵥ peut s'effectuer en une durée de 15 secondes +/- 10 secondes.

On peut noter que, au cours de l'étape (g), la volatilisation peut être suivie en contrôlant la pression dans le réacteur au moyen d'un capteur de pression. En effet, la mesure de la pression peut être un indicateur de la quantité de molécules organiques volatilisées.

Selon le procédé de la présente invention, les étapes (b) à (g) peuvent être effectuées dans une même enceinte. Aussi, le procédé de l'invention présente l'avantage d'être « one pot » (ou « un lieu »). Le procédé de la présente invention permet de réaliser l'extraction, la transformation et la volatilisation des molécules organiques présentes dans un échantillon, et ceci en un même lieu, avec un minimum d'étapes réactionnelles, en limitant l'utilisation de solvants et avec de très bons rendements. En outre, le procédé de la présente invention présente l'avantage d'être facile à mettre en oeuvre, rapide, économique et écologique.

Une fois les molécules organiques volatilisées, elles peuvent ensuite être transférées plus facilement vers un instrument d'analyse afin de les analyser. Les moyens d'analyse et les instruments d'analyse utilisables dans le cadre de l'invention sont décrits ci-dessous.

### Transfert de molécules organiques volatilisées :

Selon un mode de réalisation particulier de l'invention, le procédé de la présente invention peut comprendre en outre, après ou au cours de l'étape (g), une étape de transfert (h) des molécules organiques volatilisées vers un instrument d'analyse.

Selon l'invention, l'étape de transfert (h) des molécules organiques volatilisées peut être par exemple effectuée par entraînement au moyen d'un gaz inerte ou par aspiration. De préférence, l'étape de transfert (h) peut être réalisée par entraînement au moyen d'un balayage par un gaz inerte afin de pousser les molécules organiques volatilisées vers l'instrument d'analyse. Des gaz inertes et des moyens de balayage utilisables sont par exemple décris ci-dessous. Ainsi, les molécules organiques peuvent être directement transférées en vue de leur analyse une fois volatilisées, par exemple au fur et à mesure de leur volatilisation au cours de l'étape (g).

On peut noter que le gaz pousseur peut également entraîner les vapeurs de solvant et d'agent de fonctionnalisation introduits au cours de l'étape (f). En outre, il permet également de transférer les molécules organiques volatilisées mais non fonctionnalisées, qui peuvent être par exemple des molécules organiques volatiles également être présentes dans l'échantillon.

Le balayage par le gaz inerte peut être réalisé à la température de volatilisation Tᵥ pendant une durée Dₜ inférieure à 40 minutes, par exemple de 10 à 30 minutes.

L'étape de transfert (h) peut être réalisée à une pression Pₜ allant de 0,1 à 200 kPa, par exemple de 0,1 à 100 kPa, par exemple de 10 à 100 kPa. Des moyens permettant d'appliquer une telle pression sont décris ci-dessous.

Le balayage par le gaz inerte peut être continu. Par exemple, il peut s'agir d'un balayage avec un écoulement laminaire. Par exemple, le balayage peut être effectué à un débit allant de 1 à 10 mL/minute.

Selon un mode particulier de réalisation de l'invention, au cours de l'étape (h), les molécules organiques volatilisées peuvent être transférées vers un instrument d'analyse par chromatographie en phase gazeuse (CPG). Plus particulièrement, selon l'invention, les molécules organiques volatilisées peuvent être transférées vers un instrument d'analyse par chromatographie en phase gazeuse (CPG) couplé à un spectromètre de masse (SM) ou à un détecteur classique de CPG.

Le procédé de la présente invention peut comprendre en outre une étape d'analyse des molécules organiques volatilisées.

Le procédé de la présente invention peut être facilement mis en oeuvre, par exemple au moyen de tout dispositif approprié pour la mise en oeuvre des étapes précitées. Par exemple, le procédé de l'invention peut être mis en oeuvre au moyen d'un Dispositif de Préparation d'Echantillon (DPE) comprenant :
- un réacteur étanche comportant un moyen d'introduction d'un échantillon comprenant des molécules organiques à analyser ;
- au moins une entrée et une sortie étanches connectées audit réacteur et permettant la circulation d'un fluide de balayage au travers dudit réacteur ;
- un moyen de chauffage dudit réacteur à une vitesse moyenne de 10 à 50 °C/seconde, par exemple de 25 à 50 °C/seconde ;
- un moyen de refroidissement dudit réacteur à une vitesse moyenne de 10 à 50 °C/seconde, par exemple de 25 à 50 °C/seconde ;
- un moyen de commande et de contrôle de température dans le réacteur ; et
- un moyen de contrôle de la pression dans le réacteur ;

Le DPE présente l'avantage d'être transportable sur un site de mesure et de permettre l'analyse chimique in situ d'échantillons. Ainsi, le procédé de la présente invention peut être mis en oeuvre *in situ*, directement sur le site de mesure.

Les étapes (b) à (g) du procédé de la présente invention peuvent être réalisées dans le réacteur du dispositif décrit ci-dessus.

Par « moyen d'introduction », on entend un passage de l'extérieur vers l'intérieur du réacteur, et inversement. Il peut s'agir par exemple d'une ouverture avec un couvercle, d'un sas, d'une canalisation munie d'une vanne, d'un moyen d'injection par vis, d'un moyen d'injection par piston, etc. L'ouverture et la fermeture de ce moyen d'introduction peuvent être effectuées manuellement ou automatiquement, par exemple commandée par une unité de commande. Lorsque le moyen d'introduction est fermé, le réacteur est étanche. Le réacteur peut être par exemple constitué d'un contenant et de son couvercle. Le couvercle est tel qu'il peut fermer le réacteur de manière étanche, par exemple au moyen d'un pas de vis et d'un joint torique. Ainsi, afin d'assurer l'étanchéité du réacteur, un joint d'étanchéité peut être est disposé entre le contenant et son couvercle. Le joint est de préférence réalisé en un matériau étanche résistant à des températures supérieures à 500 °C. À titre d'exemple, le joint d'étanchéité peut notamment être réalisé en graphite, en inox et tout autre métal résistant à des températures de 500 °C.

L'entrée et la sortie étanches connectées audit réacteur permettent la circulation d'un fluide de balayage au travers du réacteur. Cette circulation, ou ce balayage du réacteur avec ledit fluide de balayage, permet par exemple d'entraîner des molécules organiques volatilisées du réacteur vers un instrument d'analyse. Ce balayage permet également d'évacuer des solvants évaporés à l'extérieur du réacteur ou encore de nettoyer et/ou de vidanger le réacteur en éliminant des impuretés volatiles susceptibles d'être présentes dans le réacteur. En particulier, ce moyen permet de mettre en oeuvre l'étape (h) du procédé de l'invention.

Le « fluide de balayage » peut être un gaz ou un liquide. Selon l'invention, le fluide de balayage peut être un gaz inerte. Le gaz inerte peut être par exemple de l'hélium, de l'azote, de l'argon, du dioxyde de carbone, etc.

Le DPE peut comprendre en outre un moyen de chauffage du fluide de balayage. Il peut par exemple s'agir d'une cartouche chauffante permettant de chauffer le fluide en sortie du réservoir ou d'une gaine thermostatée entourant la canalisation connectant ledit réservoir à l'entrée étanche du DPE. On peut par exemple citer la cartouche chauffante commercialisée par la société RS Components, France, sous la référence 731-243.

Le DPE peut comprendre en outre un moyen permettant de faire le vide ou d'appliquer une pression dans le réacteur. Ce moyen permet notamment de mettre en oeuvre l'étape (b) du procédé de l'invention. On entend par moyen permettant d'appliquer une pression dans le réacteur, tout moyen permettant de mettre l'intérieur du réacteur sous une pression de 0,1 à 2.10⁵ kPa, par exemple de 0,1 à 200 kPa, par exemple de 10 à 100 kPa. Parmi les moyens permettant d'appliquer une pression utilisable, on peut citer par exemple une pompe à vide, un générateur de pression, par exemple un compresseur pneumatique, un compresseur électrique, un compresseur à membranes, une sortie connectant le réacteur à un environnement extérieur au réacteur permettant par exemple de mettre le réacteur à la pression dudit environnement extérieur au réacteur ou à une pression intermédiaire, etc. A titre d'exemple, pour les applications spatiales sur Mars, le moyen permettant d'appliquer une pression peut être une sortie commandée par une vanne donnant sur l'environnement de Mars et permettant d'appliquer au réacteur une pression inférieure ou égale à la pression martienne qui est de 0,7 kPa. Parmi les pompes utilisables, on peut citer par exemple une pompe rotative, une pompe à piston, une pompe à palettes, une pompe à bélier, une pompe à godets, une pompe péristaltique, une pompe à vide, une pompe à engrenages, une pompe Valdès, une pompe par Airlift, une pompe hydraulique, une pompe pneumatique, ou toute autre pompe connue de l'homme du métier.

Par exemple, le moyen permettant de faire le vide ou d'appliquer une pression dans le réacteur, appelé également moyen de commande de la pression, peut permettre d'optimiser les conditions de traitement de l'échantillon, notamment les conditions d'extraction, de fonctionnalisation et de volatilisation des molécules organiques de l'échantillon. En particulier, le moyen de commande de la pression présente l'avantage d'améliorer et d'optimiser les rendements d'extraction par thermodésorption grâce à une bonne maîtrise de la pression dans le réacteur.

Le DPE peut comprendre en outre un moyen d'aspiration de fluides ou de molécules organiques volatilisées. Ce moyen permet d'évacuer par exemple les fluides issus de l'évaporation d'une substance liquide, les fluides de balayage, les fluides de refroidissement ou les molécules organiques volatilisées susceptibles d'être présentes dans le réacteur. Cette évacuation permet par exemple de vidanger ou de nettoyer le réacteur. Il peut par exemple s'agir du moyen permettant de faire le vide précité. Parmi les moyens d'aspiration utilisables, on peut citer par exemple une pompe à vide, une sortie connectant le réacteur à un environnement extérieur au réacteur dont la pression est inférieure à celle du réacteur.

Le DPE peut comprendre en outre un moyen de nettoyage du réacteur, permettant d'évacuer le solide, les fluides ou les particules susceptibles d'être présentes dans le réacteur et de nettoyer le réacteur. Par exemple, le nettoyage du réacteur peut être réalisé par pyrolyse et évacuation des impuretés volatilisées issues de la pyrolyse par un balayage par un fluide de balayage. Ou encore, le nettoyage du réacteur peut être réalisé par évacuation du solide, par exemple par le moyen d'introduction du DPE ou par un sas de vidange, puis lavage, manuel ou automatisé, avec un ou plusieurs solvant(s), comme par exemple de l'éthanol ou de l'acétone, éventuellement sous ultrasons, et éventuellement suivi d'un séchage par un gaz inerte. Avantageusement, ledit moyen de nettoyage permet le nettoyage du réacteur en moins d'une heure. Le moyen de nettoyage du DPE permet de garantir la propreté du réacteur avant ou après chaque utilisation du DPE. Par exemple, il peut permettre une grande propreté du réacteur avec une concentration d'impuretés résiduelles inférieure à 10⁻¹⁴ mole/cm³, de préférence inférieure à 10⁻¹⁵ mole/cm³. Cette propreté peut être évaluée à l'aide d'un balayage du réacteur et d'une analyse par CPG-SM ayant par exemple une limite de détection de 10⁻¹⁴ mole/cm³, par exemple de 10⁻¹⁵ mole/cm³. Ainsi, le moyen de nettoyage du DPE permet au DPE d'être réutilisable plusieurs fois.

Avantageusement, le DPE peut comprendre en outre au moins un moyen d'agitation. En effet, celui-ci peut permettre d'améliorer les rendements ou d'accélérer les étapes de préparation d'échantillon, notamment au cours des étapes d'extraction et de fonctionnalisation.

On entend par « moyen d'agitation » au sens de la présente invention tout moyen approprié pour permettre l'agitation du contenu du réacteur comprenant l'échantillon. Il peut s'agir par exemple d'un agitateur magnétique, d'un agitateur mécanique, d'une sonde à micro-ondes, d'un moyen permettant d'appliquer des ultrasons à l'intérieur du réacteur, comme par exemple une cuve à ultrasons dans laquelle est placé le DPE ou une sonde à ultrasons placée par exemple sur ou dans le réacteur. Ce moyen permet de réaliser les étapes (c) à (g) sous agitation, et notamment sous ultrasons ou micro-ondes.

Avantageusement, le moyen d'agitation peut être une sonde appliquant des ultrasons à l'intérieur du réacteur. Cette sonde résiste de préférence aux conditions de température et de pression appliquées dans le cadre de l'utilisation du DPE. Ainsi, la sonde à ultrasons peut permettre une agitation thermique d'un mélange solide-liquide ou liquide-liquide contenu dans le réacteur. Par exemple, la sonde à ultrasons peut être disposée dans un orifice du réacteur débouchant à l'intérieur de celui-ci. Il peut par exemple s'agir d'une sonde à ultrasons comprenant un générateur ultrasonique, un transducteur piézoélectrique, un adaptateur et une sonotrode. On peut par exemple citer la sonotrode commercialisée par la société Hielscher USA sous la référence UP50H. En outre, la sonde à ultrasons peut être équipée d'un variateur de fréquence et/ou d'intensité des ultrasons. Ainsi, en faisant varier la fréquence et l'intensité des ultrasons ou en optimisant les valeurs de fréquence et d'intensité des ultrasons, ladite sonde peut permettre d'améliorer le traitement, notamment l'extraction et la fonctionnalisation, de l'échantillon, en termes d'efficacité, de rapidité et/ou de rendement. Ce moyen d'agitation présente également l'avantage d'être peu encombrant et donc plus compatible avec des contraintes d'encombrement du DPE, par exemple pour être transportable facilement ou pour une utilisation extraterrestre.

Avantageusement, le DPE peut comprendre en outre au moins un moyen de rétention de l'échantillon solide dans le réacteur, agencé pour retenir l'échantillon lors de la circulation d'un fluide de balayage. Ainsi, le moyen de rétention de l'échantillon solide permet notamment d'éviter les pertes d'échantillon solide au cours du fonctionnement du dispositif, et en particulier lors du balayage du réacteur par un fluide de balayage. Ce moyen de rétention permet également d'éviter que des particules de l'échantillon solide n'atteignent le ou les instruments d'analyse.

Le moyen de rétention peut être par exemple une grille ou un matériau de protection de porosité inférieure à la granulométrie minimale de l'échantillon placée en sortie des fluides de balayage afin d'empêcher le passage du solide. Le moyen de rétention peut être par exemple une couche de fritté, de laine de verre ou de coton disposée à la sortie du réacteur pour éviter, lors du balayage par le fluide de balayage, d'entraîner une partie de l'échantillon solide. On notera, toutefois, que compte tenu des faibles pressions de gaz utilisées lors du balayage, l'utilisation d'un tel moyen de rétention n'est pas obligatoire. En outre, le moyen de rétention peut être nettoyable et/ou remplaçable en cas d'obstruation par le solide.

Avantageusement, le DPE peut comprendre en outre au moins un moyen étanche d'introduction d'une substance liquide dans le réacteur. Ce moyen permet notamment l'ajout de substance liquide au cours de l'étape (f) du procédé de l'invention.

On entend par « substance liquide » tout liquide ou mélange de liquides utilisable dans le cadre de l'application de DPE. Il peut s'agir par exemple d'un solvant, d'un mélange de solvants ou d'un réactif, comme par exemple un agent de fonctionnalisation, celui-ci étant éventuellement en mélange dans un solvant.

On entend par « moyen étanche d'introduction d'une substance liquide » tout moyen étanche connu de l'homme du métier permettant d'introduire une substance liquide dans un réacteur. Le moyen étanche d'introduction d'une substance liquide dans le réacteur peut être par exemple un septum constitué d'une matière à la fois perforable et auto-obturable. Ainsi, le septum peut être traversé par une aiguille ou une seringue permettant l'injection de ladite substance liquide. Le septum peut être constitué de toute matière perforable et auto-obturable connue de l'homme du métier, par exemple du silicone, du polyuréthane, du caoutchouc, etc. Le moyen étanche d'introduction d'une substance liquide dans le réacteur peut également être un conduit muni d'une vanne contrôlant l'introduction de ladite substance liquide dans le réacteur. La vanne peut éventuellement être connectée à une unité de commande qui permet de commander et de contrôler l'injection de substance liquide et le volume de substance liquide à injecter.

Selon un autre mode de réalisation, le moyen d'introduction d'une substance liquide peut être composé d'un compartiment, formé par exemple dans le fond du réacteur, contenant la substance liquide et séparé de l'échantillon de solide par un film de matériau perforable. Ainsi, ladite substance liquide peut être mise en contact avec l'échantillon à l'aide d'un système de perforation du film. Dans un autre mode de réalisation, le moyen d'introduction d'une substance liquide peut être composé d'une capsule contenant une substance liquide, en matériau thermodestructible et disposée dans le réacteur. Ainsi, ladite substance liquide peut être mise en contact avec l'échantillon par chauffage.

Le DPE peut comprendre en outre au moins un réservoir destiné à contenir une substance liquide, connecté audit moyen étanche d'introduction d'une substance liquide. Celui-ci permet d'approvisionner directement le réacteur en substance liquide, par exemple en agent de fonctionnalisation et/ou en solvant ajoutés au cours de l'étape (f) du procédé de l'invention. Ce réservoir peut être par exemple utile pour des applications spatiales du procédé et du dispositif décrits dans la présente.

Dans le DPE décrit ci-dessus, le moyen de contrôle de la température peut être un capteur de température positionné dans le réacteur. Le moyen de commande et de contrôle de la température peut être un thermocouple associé avec le moyen de chauffage du réacteur. Ce moyen permet de commander et contrôler l'élévation ou la diminution de température au cours des étapes (c) ou (e) et de réguler la température au cours des étapes (d), (f), et (g) du procédé de l'invention.

Dans le DPE décrit ci-dessus, le moyen de contrôle de la pression peut comprendre un capteur de pression positionné dans le réacteur, par exemple connecté à un orifice formé dans le réacteur ou déporté légèrement en aval de celui-ci, par exemple sur un conduit allant du réacteur vers un instrument d'analyse. Ainsi, la pression peut être mesurée soit directement dans le réacteur, soit à la sortie du réacteur, par exemple jusqu'à 3 cm en aval du réacteur, par exemple à 2 cm en aval du réacteur. Le capteur de pression résiste de préférence aux conditions de température et de pression appliquées dans le cadre de l'utilisation du DPE. On peut par exemple citer le capteur de pression à fibre optique commercialisé par la société FISO Technologies (Canada) sous la référence FOP-MH, qui supporte la température de 450°C. Le moyen de contrôle de la pression permet de mettre en oeuvre l'étape (b) du procédé de l'invention.

Avantageusement, le DPE de l'invention peut comprendre en outre un système d'acquisition des valeurs de température et de pression, ledit système permettant de contrôler, de suivre et d'enregistrer en continu la température et la pression dans le réacteur au cours de l'utilisation du DPE. Ce système permet ainsi d'acquérir et de traiter les données de température et de pression sur un ordinateur, et/ou, lorsqu'il est couplé à un moyen de commande de la température et/ou de la pression, de fixer ou réajuster la température et la pression à l'intérieur du DPE. Ce système permet donc de savoir ce qui se passe dans le DPE à un instant donné et d'optimiser toutes les conditions. Parmi les systèmes utilisables, on peut citer par exemple un Equipement de Test et de Contrôle (ETC), permettant l'acquisition de la température et de la pression, ainsi que leur contrôle. Cet équipement de Test et de Contrôle intègre notamment le Logiciel LabVIEW (Signal Express avec SSP), une carte d'acquisition (NI PCI-4351) de températures et de pression et une alimentation stabilisée avec une entrée pour le pilotage analogique. Cet équipement intègre également une phase de programmation de l'interface Homme / Machine et de la gestion de la régulation thermique permettant le stockage des données.

Dans le DPE décrit ci-dessus, la forme du réacteur peut être quelconque. Le réacteur peut par exemple être sous forme de disque, sous forme cylindrique, parallélépipédique, pyramidal, conique, tronconique, etc. Ce dispositif peut être en une seule partie ou en plusieurs parties, par exemple en deux parties, par exemple sous forme d'un contenant et de son couvercle.

Le réacteur est de préférence constitué d'un matériau conducteur de chaleur de façon à permettre des montées et descentes rapides de la température à l'intérieur du réacteur. Il peut s'agir de tout matériau conducteur permettant de résister aux conditions de température et de pression appliquées dans le cadre de l'utilisation du DPE. Des intervalles de température et de pression applicables dans le cadre de l'utilisation du DPE sont par exemple décrits ci-dessous. Avantageusement, le réacteur peut être constitué d'un ou plusieurs matériaux choisis dans le groupe comprenant l'inox, le cuivre, le titane, l'acier ou la céramique.

Le volume du réacteur peut être choisi suivant la quantité d'échantillon à préparer en vue de l'analyse des molécules organiques qu'il contient. Par exemple, pour un volume d'échantillon de 0,1 à 4 cm³, le volume de l'enceinte du réacteur peut être de 2 à 10 mL.

La forme du fond du réacteur peut être choisie de façon à permettre l'optimisation de la préparation de l'échantillon, par exemple en permettant le placement de l'échantillon dans un lieu précis de l'intérieur du réacteur ou en maximisant les surfaces de contact entre l'échantillon et une substance liquide introduite dans le réacteur. De préférence, la forme du fond du réacteur permet de concentrer l'échantillon au fond du réacteur et de favoriser l'immersion totale de l'échantillon dans la substance liquide. Par exemple, le fond du réacteur peut être sous forme plane, courbe, sous forme de cupule, sous forme conique, etc. En outre, la surface de l'intérieur du réacteur est de préférence lisse afin d'éviter que des poches de fluide ou de gaz subsistent après le balayage par un fluide de balayage.

Les parois internes du réacteur sont avantageusement en matériau inattaquable, en particulier lorsque les conditions d'utilisation du réacteur peuvent être corrosives. Par exemple, l'agent de fonctionnalisation utilisé au cours de l'étape (f) du procédé de l'invention peut être corrosif. Les parois internes du réacteur peuvent être par exemple en métal inattaquable ou recouvertes d'un revêtement inattaquable afin d'éviter que les substances liquides introduites dans le réacteur n'attaquent les parois du réacteur. Parmi les revêtements utilisables, on peut citer par exemple le traitement Silcosteel (marque déposée) développé par la société Restek (USA) et décrit dans le brevet US N° 6,511,760 **(ref 5).** Ce revêtement a une épaisseur de 300Å à 350Å et supporte une température maximale de 600°C.

Les moyens de chauffage et de refroidissement très rapides du DPE décrit ci-dessus peuvent permettre de maîtriser parfaitement les temps durant lesquels le réacteur est soumis aux hautes ou basses températures, puisque les paliers intermédiaires de montée ou de descente de température sont maîtrisés et atteints très rapidement. Ainsi, le DPE présente l'avantage de permettre l'extraction de molécules organiques à des températures extrêmes (par exemple à des températures supérieures à 250°C, par exemple supérieures à 350°C, par exemple supérieures à 450°C) pendant des durées bien maîtrisées, tout en évitant leur dégradation, notamment par refroidissement rapide après l'extraction.

Dans le DPE décrit ci-dessus, le moyen de chauffage dudit réacteur peut être choisi dans le groupe comprenant une cartouche chauffante, une plaque chauffante, une résistance électrique, un balayage de gaz chaud, un chauffage radiatif, par exemple par induction ou par halogène. Avantageusement, le moyen de chauffage peut être une cartouche chauffante. La cartouche chauffante peut être composée d'une résistance électrique, par exemple de forme cylindrique, éventuellement protégée par un blindage, par exemple en acier inoxydable. Afin de permettre une montée rapide de la température du réacteur, la puissance de la cartouche chauffante peut être par exemple supérieure à 400 W (Watt). On peut par exemple citer la cartouche chauffante commercialisée par la société RS Components (France) sous la référence 731-243. Le moyen de chauffage permet notamment la mise en oeuvre des étapes (c), (d), (f) et (g) du procédé de l'invention.

Dans le DPE décrit ci-dessus, le moyen de refroidissement dudit réacteur peut être choisi dans le groupe comprenant une plaque de refroidissement, un bain de liquide, par exemple de l'eau, un gel de refroidissement, un balayage du réacteur par un fluide de refroidissement, un circuit de refroidissement en contact avec le réacteur ou dans les parois du réacteur. Le moyen de refroidissement permet notamment la mise en oeuvre de l'étape (e) du procédé de l'invention.

On entend par gel ou par fluide « de refroidissement » un gel ou un fluide, par exemple un liquide ou un gaz, permettant le refroidissement rapide du réacteur à une vitesse moyenne de 10 à 50 °C/seconde, par exemple de 25 à 50 °C/seconde. Parmi les liquides ou gels de refroidissement, on peut citer par exemple l'eau, l'azote liquide, le CO₂ ou les mélanges réfrigérants comprenant par exemple de la glace ou de la carboglace. Parmi les gaz de refroidissement, on peut citer par exemple l'air froid, le CO₂, l'azote, l'argon, l'hélium, etc.

On entend par « circuit de refroidissement » un circuit comprenant un ou plusieurs conduits dans lesquels sont mis en circulation des fluides de refroidissement, par exemple des liquides ou gaz de refroidissement. Le circuit de refroidissement peut par exemple être compris dans les parois du réacteur ou dans la plaque de refroidissement.

On entend par « plaque de refroidissement » une plaque capable d'absorber la chaleur du DPE par transfert thermique. Il peut s'agir d'une plaque pleine en matériau conducteur de chaleur ou d'une plaque traversée par un circuit de refroidissement.

Avantageusement, le moyen de refroidissement dudit réacteur est une plaque de refroidissement absorbant la chaleur mise en contact direct avec le DPE. La rapidité du refroidissement du réacteur peut dépendre de divers paramètres comme par exemple le matériau conducteur de la plaque de refroidissement et/ou du DPE, l'épaisseur de la plaque de refroidissement, la surface de contact de la plaque de refroidissement avec le DPE, la qualité du contact de la plaque de refroidissement avec le DPE, le volume du DPE à refroidir, la variation de température à effectuer. Les calculs de transfert de chaleur peuvent être effectués en utilisant par exemple un coefficient d'échange H de 10 000 W/m²/°C. Parmi les matériaux conducteurs utilisables, on peut citer par exemple le cuivre, l'inox ou le titane. En outre, pour réaliser un refroidissement rapide à une vitesse moyenne de 10 à 50 °C/seconde, les dimensions de la plaque de refroidissement peuvent être par exemple, pour un volume de réacteur de 2 à 10 mL, de 20x20x10 (mm) à 80x80x40 (mm), de préférence de 35x35x15 (mm) à 45x45x25 (mm). Avantageusement, le DPE peut être maintenu sur la plaque de refroidissement avec une force de serrage de 500 à 2000 N de préférence de 900 à 1600 N afin de réaliser un bon contact et d'optimiser le refroidissement. Le mode de serrage peut par exemple être réalisé au moyen de pinces de serrage, ou d'un système de mise en pression comme par exemple une presse. Ce moyen de refroidissement présente notamment l'avantage de permettre une descente en température jusqu'à une température de - 50°C et même au-delà grâce à un balayage de la plaque froide par un fluide froid tel que de l'azote liquide ou de CO₂.

Le procédé de la présente invention peut comprendre en outre une étape d'analyse des molécules organiques extraites de l'échantillon qui les contient, fonctionnalisées et volatilisées. L'analyse de ces molécules organiques peut être réalisée par tout moyen d'analyse appropriée connue de l'homme du métier. Il peut s'agir par exemple d'un instrument d'analyse connecté en aval du DPE.

Par exemple, l'instrument d'analyse peut être connecté en aval du DPE décrit ci-dessus pour former un système d'analyse. Ce système d'analyse permet la mise en oeuvre du procédé de l'invention et l'analyse chimique des molécules organiques de l'échantillon.

On entend par « instrument d'analyse » au sens de la présente invention tout appareil permettant l'analyse chimique de molécules organiques, par exemple l'analyse moléculaire, chirale, et même isotopique (cette dernière pouvant être obtenue au moyen d'un spectromètre de masse par exemple). L'instrument d'analyse peut par exemple être choisi dans le groupe comprenant un spectromètre infra-rouge, un chromatographe, utilisant par exemple la chromatographie en phase gazeuse (CPG) ou la chromatographie liquide haute performance (CLHP), un spectromètre de masse (SM). L'instrument d'analyse peut éventuellement être un instrument miniaturisé par exemple pour des applications spatiales du système d'analyse. On peut par exemple citer l'instrument d'analyse par chromatographie en phase gazeuse décrit dans le document de Sternberg et al., Encyclopaedia of Separation Science, Academic Press, London, (2006) **(ref 6).**

Le système d'analyse peut comprendre en outre un détecteur connecté en aval dudit instrument d'analyse. Parmi les détecteurs utilisables on peut citer par exemple un spectrophotomètre, un spectromètre infra-rouge (IR), un spectromètre de masse (SM), un détecteur à ionisation de flamme (FID ou « Flame ionization detector »), un détecteur à conductivité thermique (TCD ou « Thermal Conductivity Detector »).

De préférence, l'instrument d'analyse peut être un chromatographe en phase gazeuse. De préférence, l'instrument d'analyse peut être un chromatographe en phase gazeuse (CPG) couplé à un spectromètre de masse (SM) ou à un détecteur, qui peut être un détecteur classique de CPG. On peut citer par exemple l'instrument d'analyse décrit dans le document de Sternberg et al., Encyclopaedia of Separation Science, Academic Press, London, (2006) **(ref 6).**

Le système d'analyse peut comprendre plusieurs DPE connectés ou connectables à l'instrument d'analyse. Les DPE peuvent par exemple être connectés et déconnectés un par un à l'instrument d'analyse, afin de réaliser successivement, pour chaque DPE, le traitement et l'analyse de l'échantillon qu'il contient. Les DPE peuvent être connectés et déconnectés manuellement ou automatiquement à l'instrument d'analyse à l'aide de tout moyen de connexion approprié, par exemple au moyen de canalisations munies de vannes.

Avantageusement, le système d'analyse peut comprendre en outre, en aval du DPE et en amont de l'instrument d'analyse, un moyen d'injection des molécules organiques dans l'instrument d'analyse. Il peut s'agir par exemple d'un thermodésorbeur ou d'une boucle d'échantillonnage permettant l'injection des molécules organiques par l'intermédiaire d'un gaz inerte. Cette boucle suivant son volume permet d'injecter ponctuellement des volumes de gaz et de molécules organiques allant de 10 à 2000 µL. Une telle boucle est par exemple décrite dans le document Meunier et al., Advances in Space Research (2007) 39, 3, pp 337-344 **(ref 7).** Parmi les injecteurs thermodésorbeurs utilisables, on peut citer par exemple l'injecteur OPTIC 3 commercialisé par la société ATAS GL International BV (Pays-Bas).

Le système d'analyse peut comprendre, en outre, un circuit de balayage des molécules organiques comprenant :
- un réservoir de fluide de balayage, comme par exemple une bouteille de gaz inerte ou un réacteur contenant le fluide de balayage ;
- une canalisation connectant ledit réservoir de fluide de balayage à l'entrée étanche du DPE ; et
- une canalisation connectant la sortie du DPE à l'instrument d'analyse.

Le système d'analyse peut être un système amovible, c'est-à-dire qui peut être monté, démonté et/ou déplacé ou encore dont les éléments constituant le système d'analyse peuvent être séparés. En outre, il présente l'avantage d'être facilement transportable sur un lieu de prélèvement d'échantillon.

En résumé, le DPE décrit ci-dessus présente l'avantage de permettre en un seul lieu, le réacteur, et avec un minimum d'étapes la préparation d'un échantillon et notamment l'extraction, la fonctionnalisation et la volatilisation de molécules organiques présentes dans un échantillon, ainsi que leur transfert vers un instrument d'analyse. Il facilite ainsi grandement la mise en oeuvre du procédé de l'invention destiné à préparer l'échantillon en vue de l'analyse des molécules qu'il contient. En outre, en effectuant toutes ces étapes en un même lieu, il permet d'éviter les pertes liées aux transferts de l'échantillon dans différents réacteurs et d'obtenir de bons rendements.

En outre, le DPE permet un parfait contrôle de la propreté et de la fiabilité de l'analyse de l'échantillon. En effet, une fois l'échantillon introduit, il n'y a plus de risque de pollution jusqu'à l'analyse. Il permet ainsi une meilleure fiabilité des résultats d'analyse.

Le DPE permet également l'analyse de traces dans différentes matrices et cela avec de faibles masses d'échantillons.

En outre, il peut permettre l'analyse des molécules organiques contenues dans un échantillon sans dénaturation de leurs propriétés chimiques et physiques, comme par exemple l'asymétrie ou la chiralité des molécules organiques.

Le DPE présente également l'avantage d'être multifonctionnel, et permet de réaliser divers méthodes et procédés, comprenant par exemple une ou plusieurs étapes choisies dans le groupe comprenant l'extraction, la fonctionnalisation, la volatilisation et le transfert vers un instrument d'analyse de molécules organiques présentes dans un échantillon. En outre, il peut également combiner la fonctionnalité de pyrolyseur, par exemple pour le nettoyage du réacteur ou, si nécessaire, pour réaliser des analyses complémentaires des molécules organiques ainsi dégradées. En effet, il permet de réaliser des méthodes de pyrolyse et de thermochimiolyse, par exemple jusqu'à des températures de 1000°C, par exemple jusqu'à des températures de 600 °C. Ces fonctionnalités sont en effet permises grâce au moyen de chauffage du réacteur à une vitesse moyenne de 10 à 50°C/seconde que le DPE comprend. On peut noter que la DPE présente l'avantage de ne pas se limiter à la mise en oeuvre d'une simple pyrolyse de molécules organiques, contrairement à la plupart des dispositifs de l'art antérieur.

Le DPE permet d'appliquer à l'échantillon des conditions de température et de pression extrêmes. Le DPE peut être utilisé par exemple pour des intervalles de température allant de -100°C à 1000°C, par exemple de -50°C à 600°C, par exemple de 0°C à 600°C. Par ailleurs, le DPE peut être utilisé pour des intervalles de pression allant de 0,1 à 2.10⁵ kPa, par exemple de 0,1 à 200 kPa.

Enfin, le DPE peut être compatible avec n'importe quelles techniques d'analyse en phase gazeuse et adaptable avec des techniques séparatives comme par exemple la HPLC, l'électrophorèse capillaire, etc.

D'autres avantages pourront encore apparaître à l'homme du métier à la lecture des exemples non limitatifs ci-dessous, illustrés par les figures annexées.

### Brève description des figures

- la figure 1 est une vue schématique éclatée d'un dispositif de préparation d'échantillon (DPE) selon l'invention ;
- la figure 2 est une vue schématique latérale du DPE de la figure 1 ;
- la figure 3 est une vue schématique, de dessus, du DPE de la figure 1 ;
- la figure 4 est une vue schématique en coupe transversale du DPE de la figure 1 ;
- la figure 5 est une vue schématique, en perspective, de l'élément inférieur (2) du réacteur du DPE selon l'invention ;
- la figure 6 est une vue schématique, en coupe de l'élément inférieur (2) du réacteur du DPE ;
- la figure 7 est une vue schématique, en perspective, de l'élément supérieur du réacteur du DPE ;
- la figure 8 est une vue schématique, de dessus, de l'élément supérieur du réacteur ;
- la figure 9 est une vue schématique latérale de l'élément supérieur du réacteur ; et
- la figure 10 est une vue schématique d'un système d'analyse d'échantillon selon l'invention.
- La figure 11 représente la DPE couplée à une plaque de refroidissement au moyen d'une presse : La figure 11.a) est une vue schématique latérale de l'ensemble constitué de la presse, du DPE et de la plaque froide ; La figure 11.b) est une vue schématique de dessus de l'ensemble constitué de la presse, du DPE et de la plaque froide ; La figure 11.c) est une vue schématique en perspective de l'ensemble constitué de la presse, du DPE et de la plaque froide.
- La figure 12 représente le chronogramme synthétisant les différents cycles de température en fonction du temps de l'expérience pour le procédé « un lieu / une étape ».
- La figure 13 représente le chromatogramme issu de l'analyse chromatographique couplée à la spectrométrie de masse de l'échantillon de 0,5 g de sol du désert d'Atacama après le procédé « un lieu / une étape ».

### EXEMPLES

### Exemple 1 : Exemple d'un Dispositif de Préparation d'Echantillon utilisable pour mettre en oeuvre le procédé de l'invention.

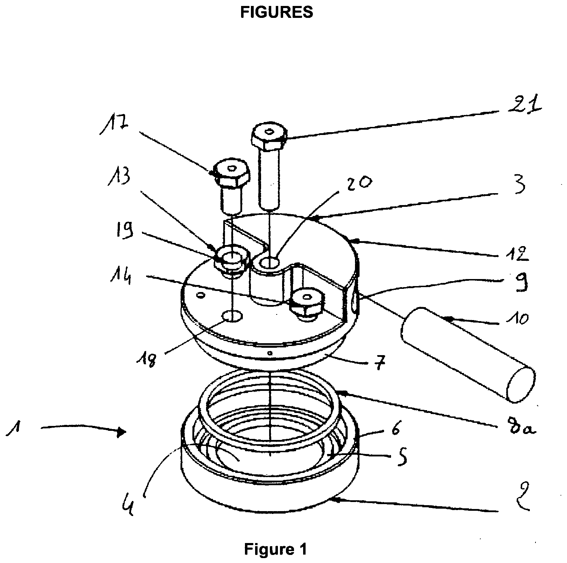

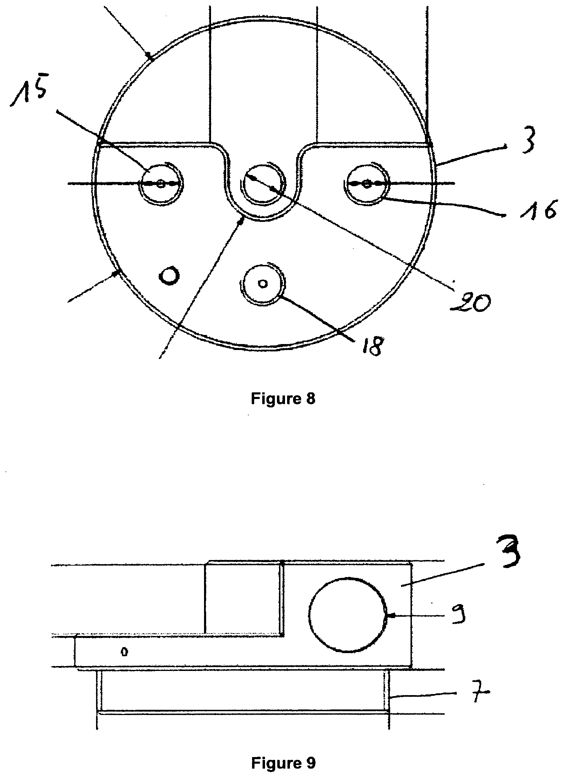

Un dispositif de préparation d'échantillon utilisable pour la mise en oeuvre du procédé de l'invention est illustré par les figures 1 à 11.

La figure 1 représente un dispositif (1) de préparation d'échantillon (DPE), en vue éclatée. Le DPE comprend un réacteur étanche comportant un élément inférieur (2) (contenant) et un élément supérieur (3) (couvercle) qui peuvent être solidarisés ou désolidarisés l'un à l'autre par vissage. Les deux éléments vissables constituent ainsi l'ouverture du réacteur permettant l'introduction d'un échantillon, non représenté, comprenant des molécules organiques à analyser.

L'élément inférieur (2) comporte un évidemment (4) destiné à recevoir l'échantillon, un épaulement circulaire (5) bordant l'évidemment (4) et une paroi latérale cylindrique (6) dont la face interne est filetée. L'élément supérieur (3) comporte une face externe cylindrique (7) qui est destinée à coopérer avec la face interne de la paroi latérale cylindrique (6) de l'élément inférieur (2) pour la solidarisation ou désolidarisation des éléments (2) et (3).

L'échantillon à analyser est introduit manuellement dans l'évidemment (4) via l'ouverture de l'élément inférieur (2) puis les éléments inférieur (2) et supérieur (3) sont solidarisés l'un à l'autre par vissage afin de former un réacteur étanche.

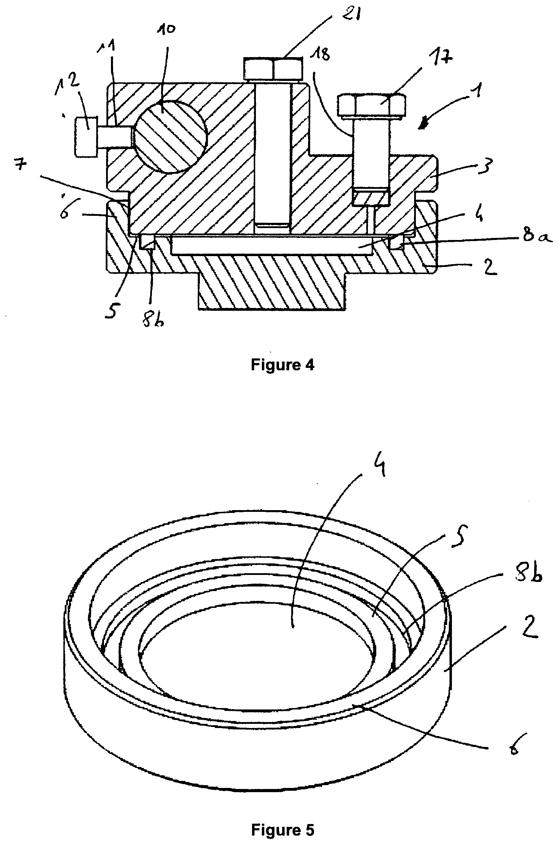

Lorsque les éléments supérieur (3) et inférieur (2) sont vissés l'un à l'autre, l'élément supérieur (3) repose sur l'épaulement circulaire (5) de l'élément inférieur. Ainsi, l'évidemment (4), obturé par l'élément supérieur (3), forme une enceinte étanche dont le volume est de 4 mL. Afin d'assurer l'étanchéité du réacteur, un joint d'étanchéité (8a) est disposé entre les éléments supérieur (3) et inférieur (2). Dans le mode de réalisation illustré (voir figure 4), une gorge (8b) annulaire de réception du joint d'étanchéité (8a) est formé dans l'épaulement (5) de l'élément inférieur (3). Le joint (8a) est réalisé en graphite.

Le réacteur est de forme cylindrique et est réalisé en titane. Plus précisément, les éléments (2), (3), (4), (5), (6) et (7) sont en titane.

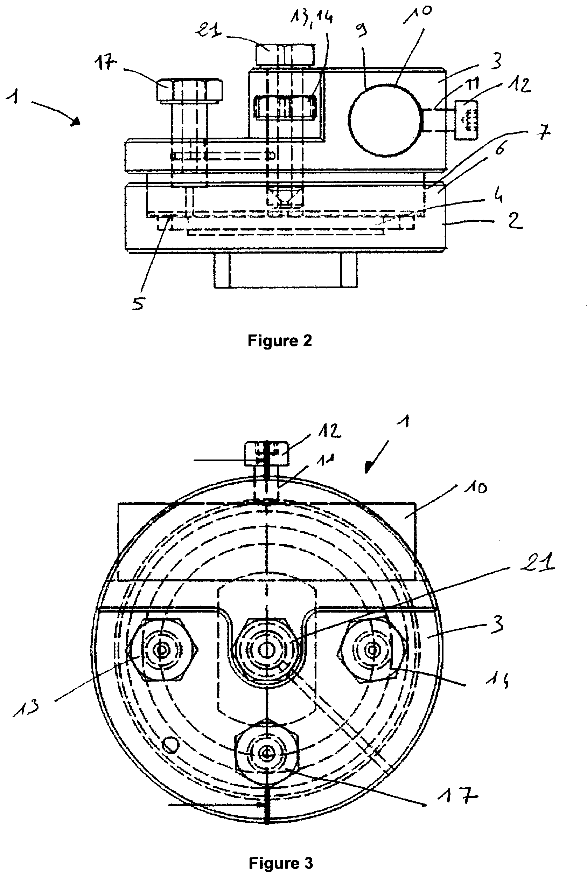

Un logement de réception (9) du moyen de chauffage (10), représenté sur les figures 2 à 4, est formé dans l'élément supérieur (3) du réacteur. Ledit logement (9) est un alésage horizontal présentant une forme cylindrique, agencé pour recevoir une cartouche chauffante amovible (10). Un orifice fileté (11) débouchant dans le logement (9) est formé dans l'élément supérieur (3). L'orifice (11) permet l'introduction d'une vis filetée (12) dont l'extrémité est destinée à coopérer avec la cartouche chauffante (10) afin de la maintenir en position dans le logement (9).

La cartouche chauffante (10) est composée d'une résistance électrique de forme cylindrique protégé par un blindage, en acier inoxydable. La puissance de la cartouche chauffante est supérieure à 400 W, permettant une montée rapide de la température du réacteur.

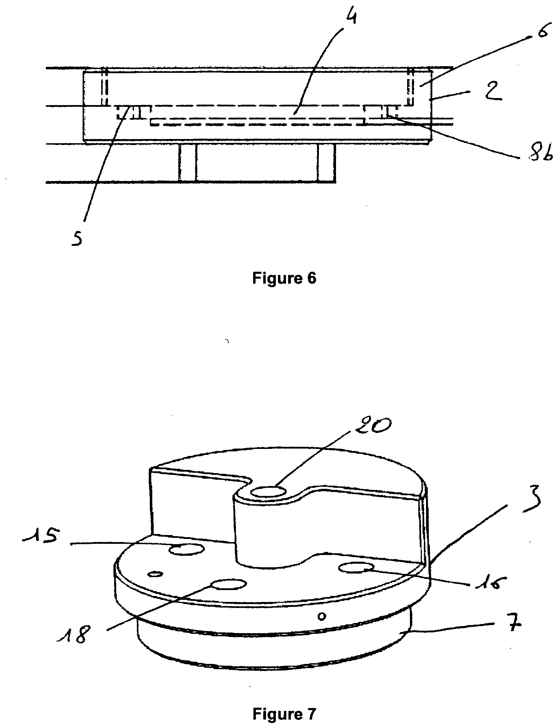

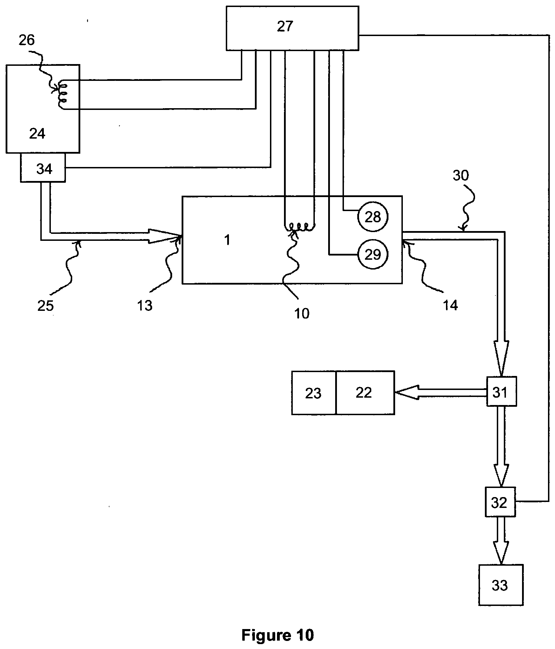

Par ailleurs, le moyen de chauffage (10) est connecté à une unité de commande (27) (voir figure 10). En outre, le dispositif (1) comprend un capteur de température (28), du type thermocouple, connecté à l'unité de commande (27) afin de réguler la température dans l'enceinte du réacteur. Le capteur de température (28) est agencé pour mesurer précisément la température, au dixième ou au centième de degrés. Il est localisé dans l'élément supérieure (3) du DPE dans un orifice fileté (20) (figure 7).

Le dispositif (1) comprend également un capteur de pression (29) à l'intérieur de l'enceinte du réacteur. Le capteur de pression sert notamment d'indicateur de la volatilisation des molécules organiques contenues dans l'échantillon. Dans le mode de réalisation représenté sur les figures 1 à 4, une vis centrale (21) coopère avec un orifice filetée (20) débouchant dans l'enceinte du réacteur. La vis centrale (21) comprend un alésage central permettant l'introduction du capteur de pression (29) ou la réalisation d'une prise de pression. Le capteur de pression (29) est également connecté à l'unité de commande (27).

Le dispositif de préparation d'échantillon (1) est également équipé d'un moyen de refroidissement (35) dudit réacteur à une vitesse moyenne de 10 à 50 °C/seconde. Dans un mode de réalisation, le moyen de refroidissement (35) est composé d'un bain d'eau apte à recevoir le réacteur.

Par ailleurs, le dispositif de préparation d'échantillon (1) comprend une entrée (13) et une sortie (14) étanches, connectées audit réacteur, et permettant la circulation d'un fluide de balayage au travers dudit réacteur. À cet effet, l'élément supérieur comprend deux orifices (15), (16) filetés débouchant à l'intérieur de l'enceinte du réacteur, à deux extrémité de celle-ci. Chacun desdits orifices (15), (16) coopère avec une vis filetée à tête hexagonale. Lesdites vis filetées sont pourvues d'un alésage central permettant le passage du fluide de balayage et sont respectivement destinées à coopérer avec une canalisation d'amenée (25) du fluide de balayage et une canalisation d'évacuation (30) dudit fluide de balayage.

En outre, le dispositif de préparation d'échantillon comprend un moyen étanche d'introduction d'une substance liquide. Dans le mode de réalisation représenté sur les figures 1 à 4, ledit moyen d'introduction de la substance liquide comprend un orifice fileté (18), formée dans l'élément supérieur du réacteur et débouchant à l'intérieur de l'enceinte, et une vis filetée (17) à tête hexagonale, munie d'un septum (19). La vis filetée (17), pourvue d'un alésage central et munie du septum (19) est introduite dans ledit orifice (18). Ainsi, la substance liquide peut être injectée au moyen d'une aiguille apte à perforer le septum, par exemple une seringue contenant ladite substance liquide.

Afin de faciliter l'extraction des molécules organiques contenues dans l'échantillon à faible température et sur un temps court, le dispositif de préparation d'échantillon est équipé d'un moyen d'agitation dans le réacteur. À cet effet, dans le présent mode de réalisation, le dispositif de préparation d'échantillon comprend une cuve à ultrasons dans laquelle le réacteur peut être placé.

On peut noter qu'un dispositif utilisable ne se limite pas à un réacteur en deux parties et l'on peut prévoir d'autres types d'ouvertures permettant l'introduction de l'échantillon à l'intérieur du réacteur. On peut notamment prévoir une ouverture formée dans le corps d'un réacteur monobloc, équipée d'un moyen amovible de fermeture. En outre, dans un mode de réalisation non illustré, l'ouverture est équipée d'un sas permettent le passage de l'échantillon entre le milieu extérieur et l'enceinte du réacteur. Ce mode de réalisation est particulièrement avantageux dans le cadre d'une utilisation spatiale.

Par ailleurs, on peut également prévoir un dispositif automatique d'introduction de l'échantillon à l'intérieur de l'enceinte.

### Exemple 2 : Dispositif de Préparation d'Echantillon couplé à une plaque de refroidissement.

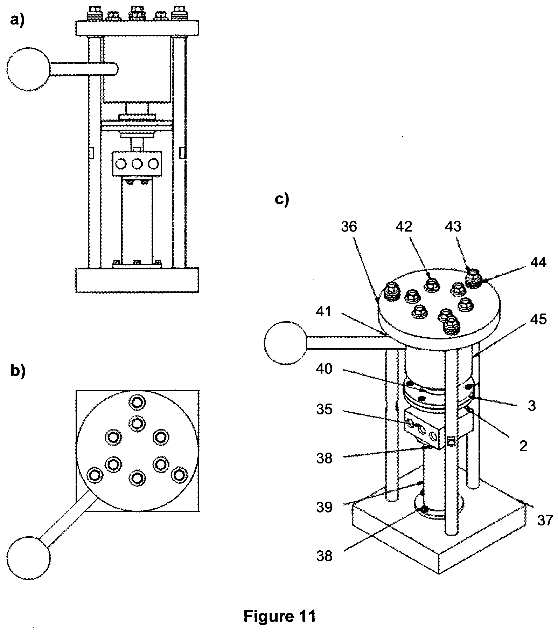

Un autre DPE a été réalisé et comporte les caractéristiques et éléments du DPE de l'exemple 1 mais avec un moyen de refroidissement différent. Dans ce second mode de réalisation, le moyen de refroidissement (35) dudit réacteur à une vitesse moyenne de 10 à 50 °C/seconde est une plaque de refroidissement en Inox (60x60x20 mm) contactée au réacteur au moyen d'une presse (Figure 11). En outre, cette plaque permet une descente en température jusqu'à -50°C grâce à une circulation d'un flux froid dans une double enveloppe ou grâce à un flux d'air froid (CO₂).

### Exemple 3: Système d'analyse comprenant un Dispositif de Préparation d'Echantillon selon l'invention

Un système d'analyse selon l'invention a été réalisé avec le DPE de l'exemple 1 et est représenté sur la figure 10.

Le système d'analyse comprend le dispositif de préparation d'échantillon (1), selon l'invention, connecté à un chromatographe en phase gazeuse (22), couplé à un spectromètre de masse (23).

Afin de permettre l'acheminement des molécules organiques volatilisées à analyser vers l'instrument d'analyse (22), le système est équipé d'un circuit de balayage comprenant un réservoir (24) de gaz inerte, une première canalisation (25) connectant le réservoir (24) à l'entrée (13) du dispositif de préparation d'échantillon (1) via une vanne (34) et une seconde canalisation (30) connectant la sortie (14) du DPE (1) à l'instrument d'analyse (22).

Le réservoir (24) est équipé d'un moyen de chauffage (26) du gaz inerte connecté à l'unité de commande (27).

Par ailleurs, le système d'analyse est équipé d'un moyen permettant le nettoyage et la vidange du dispositif. À cet effet, la canalisation de sortie (30) est équipée d'une vanne (31) connectée à l'unité de commande (27) et permettant de basculer le flux, soit vers l'entrée de l'instrument d'analyse (22), soit vers une pompe (32) dirigeant le flux vers un récipient d'évacuation (32). Ainsi, afin de nettoyer le réacteur par balayage après analyse ou d'évacuer les solvants évaporés après l'extraction, un gaz inerte est injecté via la première canalisation (25) puis aspiré par la pompe (32) afin d'évacuer les impuretés vers le récipient d'évacuation (33).

En outre, la pompe (32) connectée à l'unité de commande (27) permet de réguler la pression à l'intérieur de l'enceinte du réacteur et permet notamment de faire le vide à l'intérieur de celle-ci.

Le DPE et le système d'analyse sont illustrés par les figures 1 à 11, sur lesquelles la signification des numéros est la suivante :
1 : dispositif de préparation de l'échantillon (DPE)
2 : élément inférieur du DPE
3 : élément supérieur du DPE
4 : évidemment de l'élément inférieur (2)
5 : épaulement circulaire (5) bordant l'évidemment (4)
6 : paroi latérale cylindrique
7 : face externe cylindrique de l'élément supérieur (3)
8a : joint d'étanchéité
8b : gorge annulaire de réception du joint d'étanchéité (8)
9 : logement de réception du moyen de chauffage (10)
10 : cartouche chauffante amovible
11 : orifice fileté débouchant dans le logement (9)
12 : vis filetée introduite dans l'orifice (11) destinée à maintenir la cartouche chauffante (10)
13 : entrée de la DPE connectée au réservoir (24) de gaz inerte par une canalisation (25) munie d'une vanne (34)
14 : sortie de la DPE connectée à une évacuation (30), le flux gazeux pouvant être ensuite redirigé soit vers l'instrument d'analyse (22), soit vers un récipient d'évacuation (32).
15, 16 : orifices filetés débouchant à l'intérieur de l'enceinte du réacteur coopérant chacun avec une vis filetée à tête hexagonale
17 : vis filetée (17) à tête hexagonale introduite dans l'orifice (18)
18 : orifice fileté (18) débouchant à l'intérieur de l'enceinte
19 : septum coopérant avec l'orifice fileté (18) et la vis filetée (17)
20 : orifice fileté débouchant dans l'enceinte du réacteur
21 : vis centrale coopérant avec un orifice filetée (20) et comprenant un alésage central permettant l'introduction du capteur de pression (29)
22 : chromatographe en phase gazeuse
23 : spectromètre de masse
24 : réservoir de gaz inerte
25 : canalisation d'amenée du gaz inerte
27 : unité de commande
28 : capteur de température, du type thermocouple
29 : capteur de pression connecté à l'unité de commande (27)
30 : canalisation d'évacuation du gaz inerte munie d'une vanne (31)
31 : vanne de la canalisation (30) connectée à l'unité de commande (27) permettant de basculer le flux gazeux soit vers l'entrée de l'instrument d'analyse (22), soit vers une pompe (32) dirigeant le flux vers un récipient d'évacuation
32 : pompe permettant d'évacuer le flux gazeux vers un récipient d'évacuation
33 : récipient d'évacuation
34 : vanne de la canalisation (25) reliant l'entrée du DPE (13) au réservoir de gaz inerte (24)
35 : moyen de refroidissement (plaque froide ou bain d'eau)
36 : élément supérieur de la presse
37 : élément inférieur de la presse
38 : vis
39 : support de la plaque
40 : rotor
41 : tige support
42 : vis
43 : vis
44 : rondelles Belleville
45 : vérin de mise en pression

### Exemple 4 : Exemple de fabrication du DPE

Le DPE a été réalisé par moulage. Le métal en fusion est coulé pour remplir deux moules l'un ayant la forme de l'élément inférieur (2) du DPE et l'autre ayant la forme de l'élément supérieur (3) du DPE. Dans cet exemple, le DPE est fabriqué à partir d'inox 316L ou de titane selon un usinage traditionnel à pression atmosphérique (c'est-à-dire à une pression de 100 kPa +/- 5 kPa) et à température ambiante (c'est à dire à une température allant de 20 à 25°C).

### Exemple 5 : Exemple de procédé de fonctionnalisation extractive de molécules organiques : procédé « un lieu - une étape » (ou « one pot - one step »)

Dans cet exemple, un procédé d'extraction, de fonctionnalisation et de volatilisation de molécules organiques contenues dans un échantillon solide est réalisé à l'aide du DPE de l'exemple 1.

L'analyse d'un échantillon de sol du désert d'Atacama au Chili (analogue de sol martien) est effectuée suivant le protocole « one pot - one step » décrit ci-dessous.

L'utilisation d'un sol parfaitement caractérisé (sol Atacama) sert ici de référence pour comparer les performances du procédé avec celles des méthodes d'extraction classiques de laboratoire. L'objectif est de démontrer la faisabilité de l'extraction et de la fonctionnalisation des molécules organiques contenues à l'état de traces dans un échantillon de sol. Les rendements et performances du procédé sont évaluées par l'analyse CPG-SM en aval.

### Protocole :

Les étapes E1 à E6 suivantes sont réalisées successivement.

### E1. Nettoyage et conditionnement du DPE :

Un état de propreté en deçà de la limite de sensibilité du CPG-SM a été établie à l'intérieur du réacteur du DPE afin de s'assurer que le seuil de résidus présents dans le DPE ne perturbera pas la détection chimique réalisée par le CPG-SM.

La limite de propreté est estimée à 10⁻¹⁵ mole pour un volume du DPE de 4 mL.

Notons que cette fonction n'est pas utile si le réacteur est dans un état de propreté satisfaisant avant introduction de l'échantillon (comme c'est le cas d'une enceinte utilisable une seule fois par exemple).

Le conditionnement est réalisé simultanément par chauffage et balayage par un gaz inerte de l'intérieur du DPE comme suit :
- la température du réacteur est élevée de 20°C (température du laboratoire) à 500°C en 15 secondes.
- la température du réacteur est maintenue à 500°C et un balayage par de l'hélium est effectué pendant 10 minutes afin d'effectuer une pyrolyse et d'éliminer simultanément les résidus de pyrolyse par entraînement au moyen du gaz inerte. Les caractéristiques du balayage sont les suivantes :

| **Critères** | **Caractéristiques** |
|---|---|
| Gaz inerte | hélium |
| Caractéristiques de propreté du gaz | Alpha 2 |
| Débit de gaz | 10 ml/min |
| Stabilité du débit | ± 10 % (afin d'éliminer les résidus de pyrolyse) |
| Température du gaz à l'entrée | 500°C (afin d'éviter la chute de la température de pyrolyse) |
| Caractéristiques de l'entrée de gaz | 10 mL/min |
| Caractéristiques de la sortie de gaz | 10 mL/min |
| Espace balayé | Intérieur du réacteur |
| Durée du balayage | 10 min |
| Caractéristiques physiques du dispositif d'introduction du gaz | Raccord standard étanche |
| Caractéristiques physiques du dispositif de rejet du gaz | Raccord standard étanche sans filtrage |

Afin de vérifier la propreté de l'appareil après la pyrolyse et la non présence de résidus ou de molécules organiques dans le réacteur, un test à vide peut être effectué. Ce test consiste à exécuter les étapes E3 à E6 décrites ci-après mais sans échantillon.

Les conditions de propreté requises au cours des différentes opérations suivantes sont données par la limite de détection du CPG-MS qui est de 10⁻¹⁵ mole.

Par ailleurs, les conditions de propreté requises pour l'analyse d'échantillons requièrent d'avoir un dispositif d'introduction d'échantillon respectant ce critère de propreté de 10⁻¹⁵ mole. Dans cet exemple, l'étape suivante d'introduction d'échantillon a été réalisée manuellement sous une hotte à flux laminaire.

### E2. Introduction de l'échantillon :

L'échantillon à analyser est introduit manuellement, à l'aide d'une spatule métallique, au centre de l'évidemment (4) de l'élément inférieur (2) de façon à recouvrir la totalité de la surface de ce dernier. Cela permet de maximiser les surfaces de contact entre l'échantillon et les réactifs. Les caractéristiques de l'échantillon (Atacama, Chili) sont les suivantes:

| **Critères** | **Caractéristiques** |
|---|---|
| Composition chimique de l'échantillon | sable contenant des molécules organiques à l'état de trace |
| Granulométrie de l'échantillon | 100 à 250µm |
| Masse de l'échantillon | 0,5 g |
| Volume de l'échantillon | environ 0,3 mL |
| Propriétés et forme de l'échantillon | Solide et sec |
| Caractéristiques du lieu de dépôt de l'échantillon au sein du dispositif | - Dépôt uniforme pour assurer une bonne mouillabilité |
| | - Reproductibilité du dépôt |
| Condition de propreté requise au cours de cette opération | - Limite de détection du CPG-MS : 10⁻¹⁵ mole |
| | - Recours à un dispositif d'introduction respectant ce critère |
| Température du réacteur en début d'introduction | Ambiante (25 °C) |

Une fois l'échantillon introduit, le réacteur est refermé. La température et la pression de l'échantillon sont contrôlées et sont respectivement de 25°C et de 1 bar.

### E3. Extraction par thermo-désorption :

### Mise sous pression de l'échantillon

Après l'introduction de l'échantillon dans l'enceinte du réacteur, une pression de 4 mbar +/- 3 mbar est appliquée.

### Elévation de la température de l'échantillon

La température du réacteur est ensuite élevée à la température T_{d} de 500°C +/- 5°C en 15 secondes au moyen de la cartouche chauffante (10).

### Maintien de la température

La température du réacteur est ensuite maintenue à la température T_{d} de 500°C +/- 5°C pendant 5 minutes afin de désorber par thermo-désorption les molécules organiques contenues dans l'échantillon solide.

On peut noter que la température à l'intérieur de l'enceinte est régulée à l'aide de moyens de contrôle et de commande

### E4. Conditionnement du réacteur du DPE pour la fonctionnalisation des molécules organiques extraites :

### Diminution de la température

Un refroidissement est effectué afin d'obtenir une descente rapide de la température. Dans le cadre de cet exemple, la température est diminuée en 15 secondes de la température T_{d} à la température T_{f} de 75°C +/- 1°C à l'aide du moyen de refroidissement (35). Dans une réalisation, le moyen de refroidissement est un bain d'eau froide, dans une autre réalisation, le moyen de refroidissement est une plaque froide telle que décrite dans l'exemple 2.

### Maintient de la température

L'étape de diminution de la température est suivie d'une étape de stabilisation de la température afin de laisser l'échantillon atteindre la température T_{f} pour la réaction de fonctionnalisation. Ici, la température T_{f} est maintenue pendant une durée de 5 minutes.

La température à l'intérieur de l'enceinte est régulée à l'aide de moyens de contrôle et de commande.

### E5. Fonctionnalisation des molécules organiques extraites :

Une solution comprenant un agent de fonctionnalisation à température ambiante (20 °C) est introduite dans le réacteur au moyen d'une seringue par l'entrée (17) munie d'un septum (19).

La solution utilisée ici est obtenue en mélangeant 30 µL de MTBSTFA commercialisé sous la référence 19915 par la société Sigma-Aldrich (France) et 10 µL de DMF commercialisé sous la référence 227056 par la société Sigma-Aldrich (France).

Les caractéristiques et quantités de solution d'agent de fonctionnalisation introduites sont les suivantes :

| Solution d'agent de fonctionnalisation | Une solution de MTBSTFA pur (30 µL) dans le DMF pur (10 µL) |
|---|---|
| Volume total de solution introduit | 40 µL |

La température du réacteur est ensuite maintenue à la température T_{f} de 75°C +/- 5°C.

Puis, la température T_{f} est maintenue pendant une durée de 30 minutes +/- 1 minute afin de réaliser la réaction de fonctionnalisation des molécules organiques extraites.

On peut noter que la température à l'intérieur de l'enceinte est régulée à l'aide de moyens de contrôle et de commande qui permettent de mesurer la température à l'intérieur de l'enceinte avec une précision de +/-1°C et d'ajuster la température.

### E6. Volatilisation des molécules organiques fonctionnalisées :

La température du réacteur est ensuite élevée à la température Tᵥ de 200°C +/- 5°C en une durée de 15 secondes +/- 10 secondes au moyen de la cartouche chauffante (10). La température à l'intérieur de l'enceinte est régulée à l'aide de moyens de contrôle et de commande.

Puis, la température Tᵥ est maintenue pendant une durée de 10 minutes afin de réaliser la volatilisation des molécules organiques.

Simultanément, un balayage de l'enceinte par un gaz inerte est effectué au moyen de l'entrée (13) et de la sortie (14) permettant la circulation du gaz afin de transférer les molécules organiques volatilisées vers un instrument d'analyse CPG-SM. Les caractéristiques du balayage sont les suivantes :

| Gaz inerte utilisé pour le balayage (gaz pousseur) | Hélium alpha 2 |
|---|---|
| Caractéristique du flux en entrée | |
| - balayage : | Balayage continu |
| - débit | 1 mL/min |
| Caractéristique du flux à l'intérieur | Balayage continu |
| Caractéristique de l'écoulement | |
| - type de flux | Laminaire |
| - débit | 1 mL/min |
| - pression | 1 bar |
| - volume | 1 mL |

Eventuellement, une grille de protection placée en sortie du gaz permet de conserver l'échantillon à l'intérieur du réacteur.

On peut noter que toutes les molécules volatilisées présentes dans le réacteur, qu'elles soient fonctionnalisées ou non, peuvent être transférées vers l'instrument d'analyse. Par exemple, les restes de solvant et/ou d'agent de fonctionnalisation utilisés au cours de l'étape de fonctionnalisation peuvent être volatilisés en même temps que les molécules organiques fonctionnalisées et transférée.

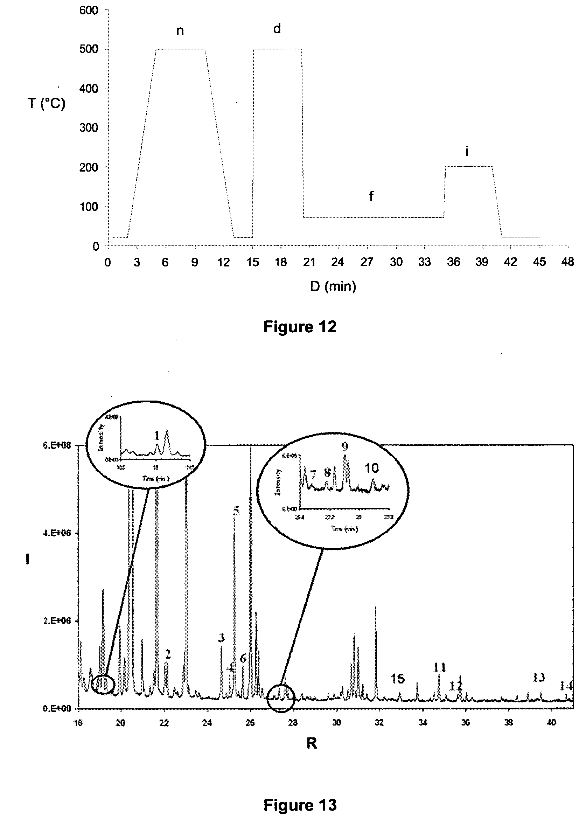

L'ensemble des étapes de cet exemple peut être illustré par la figure 12 qui représente le chronogramme synthétisant les différents cycles de température en fonction du temps de l'expérience pour l'expérimentation « un lieu / une étape » (ou « one pot / one step »). Les étapes (n), (d), (f) et (i) représentent respectivement les étapes de nettoyage du réacteur, de thermodésorption des molécules organiques, de fonctionnalisation des molécules organiques désorbées, et d'injection des molécules organiques volatilisées dans l'instrument d'analyse.

### Résultats :

La figure 13 représente le chromatogramme issu de l'analyse chromatographique couplé à la spectrométrie de masse de l'échantillon de 0,5 g de sol du désert d'Atacama après le procédé « un lieu / une étape » (ou « one pot / one step »), c'est-à-dire après chauffage à 500°C durant 5 minutes, traitement par un mélange MTBSTFA / DMF (75°C, 15 minutes) et volatilisation.

Le chromatogramme de la figure 13 représente l'évolution de l'intensité 1 du signal du détecteur, qui est liée à la concentration instantanée de la molécule organique éluée, en fonction du temps de rétention R (en minute). Sur cette représentation graphique, des pics numérotés émergent et correspondent aux molécules organiques, fonctionalisée par le MTBSTFA, suivantes : 1 : acide acetique, 2 : acide propanoïque, 3: acide benzoïque, 4: acide heptanoïque, 5 : acide octanoïque, 6: acide 4-methyl pentanoïque, 7 : alanine, 8 : glycine, 9: acide nonanoïque, 10: valine, 11: acide dodecanoïque, 12: acide phosphorique, 13: acide 1,2 benzendicarboxylique, 14: acide pentandecanoïque, 15 : 1,2 benzendicarboxylate bis(2-méthylpropyl).

En conclusion, le tableau suivant présente tous les composés organiques détectés (D) ou non (ND) dans l'échantillon de 0,5 g de sol d'Atacama (Chili) en utilisant la technique « one pot - one step ».

**Tableau 2**

| **Composés organiques contenu dans le sol du désert d'Atacama (Chili)** | **Procédure "one pot** - **one step"** |
|---|---|
| Acide benzoïque | D |
| Acide acétique | D |
| Alanine | D |
| Glycine | D |
| Acide sulphurique | ND |
| Acide nonanoïque | D |
| Valine | D |
| Leucine | ND |
| Proline | ND |
| Acide 1,2-benzendicarboxylique | D |
| Acide Phosphorique | D |
| Benzendicarboxylate bis(2-methylpropyl) | D |
| Serine | ND |
| Phenylalanine | ND |
| Acide dodecanoïque | D |
| Acide tétradécanoïque | ND |
| Acide pentadécanoïque | D |
| Acide glutamique | ND |
| Acide octadecanoïque | D |
| Benzendicarboxylate bis(2-methylpropyl) | D |

Les résultats qualitatifs obtenues à partir de la procédure "one pot - one step" ont permis de retrouver la majorité des acides carboxyliques contenus dans le sol d'Atacama. Une partie des acides aminés (les plus légers) ont pu être détectés. Cette technique a l'avantage de ne nécessiter aucun solvant et d'être réalisable en une seule étape. Ces résultats montrent que le procédé de l'invention permet d'extraire et de détecter plus de 65 % des molécules présentes dans l'échantillon. En outre, le procédé et le dispositif décrits dans la présente sont parfaitement adaptés à l'analyse *in situ.*

### Liste des références

(1) Dadkha Ali et al., Hot water extraction with in situ wet oxidation : PAHs removal from soil, Journal of hazardous materials, 2002, 93(3), 307-320.
(2) Mowry Curtis et al., Rapid detection of bacteria with miniaturized pyrolysis-gas chromatographic analysis, Proceeding of SPIE, 2001, Vol. 4575, pp. 83-90.
(3) A. Buch, D.P. Glavin, R. Sternberg, C. Rodier, C. Szopa, A new extraction technique for in situ analyses of amino and carboxylic acids on Mars by gas chromatography mass spectrometry, Planetary and Space Science, Special Astrobiology Issue, 54, 15, (2006), 1592-1599.
(4) Buch A., Marchetti C., Meunier D., Sternberg R., Raulin F., Solvent extraction of organic molecules of exobiological interest for in situ analysis of the Martian soil, Journal of Chromatography A. 999 (2003) 165-174.
(5) Brevet US N° 6,511,760.
(6) R. Sternberg, A. Buch, C. Szopa, C. Vidal-Madjar, F. Raulin, Gas chromatography in space exploration, Encyclopaedia of Separation Science, Academic Press, London, (Edition: I.D. Wilson, E.R. Adlard, M. Cooke, C.F. Poole) (2006).
(7) D. Meunier, R. Sternberg, A. Buch, C. Szopa, C. Rodier, M. Cabane, F. Raulin, A laboratory pilot for in situ analysis of refractory organic matter in Martian soil by gas chromatography-mass spectrometry. Advances in Space Research (2007) 39, 3, 337-344.

## Revendications

1. Procédé d'extraction, de fonctionnalisation et de volatilisation de molécules organiques non-volatiles présentes dans un milieu solide comprenant les étapes suivantes :
(a) prélever un échantillon solide de sol ;
(b) mettre l'échantillon sous une pression P_{d} de 0,1 à 200 kPa ;
(c) élever en moins de 30 secondes la température de l'échantillon de la température de l'environnement à une température T_{d} allant de 250°C à 600°C ;
(d) maintenir l'échantillon à la température T_{d} pendant une durée D_{d} inférieure à 15 minutes ;
(e) diminuer en moins de 30 secondes la température de l'échantillon de la température T_{d} à la température T_{f} allant de 50°C à 100°C ;
(f) ajouter à l'échantillon au moins un agent de fonctionnalisation et au moins un solvant, et maintenir l'échantillon à la température T_{f} pendant une durée D_{f} inférieure à 1 heure ; et
(g) chauffer l'échantillon à une température Tᵥ allant de 140°C à 300°C pendant une durée Dᵥ inférieure à 1 heure ce qui provoque une volatilisation des molécules organiques fonctionnalisés extraites de l'échantillon de sol.

2. Procédé selon la revendication 1, dans lequel au moins une des étapes (c) à (g) est réalisée sous ultrasons ou sous micro-ondes.

3. Procédé selon la revendication 1 ou 2, dans lequel au cours des étapes (c), (d) et (e), la température T_{d} est de 500°C +/- 10°C.

4. Procédé selon l'une quelconque des revendications 1 à 3, dans lequel au cours de l'étape (d), la température T_{d} est maintenue pendant une durée D_{d} inférieure à 5 minutes.

5. Procédé selon l'une quelconque des revendications 1 à 4, dans lequel au cours des étapes (e) et (f), la température T_{f} est de 75°C +/-10°C.

6. Procédé selon l'une quelconque des revendications 1 à 5, dans lequel, au cours de l'étape (f), l'agent de fonctionnalisation est choisi dans le groupe comprenant le *N*-méthyl-*N*-[tert-butyldiméthyl-silyl]trifluoroacétamide (MTBSTFA), le *N*,*N*-diméthylformamide diméthylacétal (DMF-DMA), l'hydroxyde de tétraméthylammonium (TMAH), l'anhydride trifluoroacétique (TFAA), l'anhydride heptafluorobutyrique (HFBA), le 2,2,2-trifluoroéthanol (TFE), le 2,2,3,3,4,4,4-heptafluoro-1-butanol (HFB), le chloroformate de méthyle (MCF), le chloroformate d'ethyle (ECF) ou un mélange de ceux-ci.

7. Procédé selon l'une quelconque des revendications 1 à 6, dans lequel, au cours de l'étape (f), le solvant est choisi dans le groupe comprenant l'eau, les alcanes en C₁ à C₈, linéaires, ramifiés ou cycliques, les alcools R^{S}-OH où R^{S} est un radical alkyle en C₁ à C₈, les cétones R^{S1}-C(=O)-R^{S2}, les éthers R^{S1}-O-R^{S2} où R^{S1} et R^{S2} sont indépendamment des radicaux alkyles en C₁ à C₈, formant éventuellement un cycle, l'isopropanol, le tétrahydrofurane (THF), le dioxane, l'acétonitrile, le diméthylformamide (DMF), le diéthylformamide, le diméthylsulfoxyde, le toluène, l'acide acétique, l'acide formique, le dichlorométhane, le chloroforme, le dichloroéthane, l'acétate d'éthyle, ou un mélange de ceux-ci.

8. Procédé selon l'une quelconque des revendications 1 à 7, dans lequel, au cours de l'étape (f), la température T_{f} est maintenue pendant une durée D_{f} de 10 à 30 minutes.

9. Procédé selon l'une quelconque des revendications 1 à 8, dans lequel, au cours de l'étape (g), la température Tᵥ est atteinte en moins de 30 secondes.

10. Procédé selon l'une quelconque des revendications 1 à 9, dans lequel, au cours de l'étape (g), la température Tᵥ est de 200°C +/- 20°C.

11. Procédé selon l'une quelconque des revendications 1 à 10, dans lequel, au cours de l'étape (g), la température Tᵥ est maintenue pendant une durée Dᵥ de 10 à 30 minutes.

12. Procédé selon l'une quelconque des revendications 1 à 11 comprenant en outre, après ou au cours de l'étape (g), une étape de transfert (h) des molécules organiques volatilisées vers un instrument d'analyse.

13. Procédé selon la revendication 12, dans lequel l'étape de transfert (h) des molécules organiques volatilisées est effectuée par entraînement au moyen d'un gaz inerte ou par aspiration.

14. Procédé selon la revendication 12 ou 13, dans lequel les molécules organiques volatilisées sont transférées vers un instrument d'analyse par chromatographie en phase gazeuse (CPG).

15. Procédé selon l'une quelconque des revendications 11 à 14, dans lequel les molécules organiques volatilisées sont transférées vers un instrument d'analyse par chromatographie en phase gazeuse (CPG) couplé à un spectromètre de masse (SM) ou à un détecteur classique de CPG.

## Patentansprüche

1. Verfahren zur Extraktion, Funktionalisierung und Verflüchtigung von nicht-flüchtigen organischen Molekülen, die in einem festen Medium, vorhanden sind, umfassend die Schritte:
(a) Entnahme einer festen Bodenprobe;
(b) Platzieren der Probe unter einem Druck P_{d} von 0,1 bis 200 kPa;
(c) Erhöhen der Temperatur der Probe von der Umgebungstemperatur auf eine Temperatur T_{d} von 250°C bis 600 °C in weniger als 30 Sekunden ;
(d) Beibehalten der Temperatur T_{d} der Probe für eine Dauer D_{d} von weniger als 15 Minuten;
(e) Senken der Probentemperatur T_{d} auf die Temperatur T_{f} von 50 °C bis 100°C
in weniger als 30 Sekunden;
(f) Zugabe zu der Probe von mindestens einem Funktionalisierungsmittel und mindestens einem Lösungsmittel und Beibehalten der Temperatur T_{f} der Probe für eine Dauer D_{f} von weniger als 1 Stunde; und
(g) Erhitzen der Probe auf eine Temperatur Tᵥ von 140°C bis 300 °C für eine Dauer Dᵥ von weniger als 1 Stunde, was eine Verflüchtigung der funktionalisierten, organischen, aus der Bodenprobe extrahierten Moleküle verursacht.

2. Verfahren nach Anspruch 1, wobei mindestens einer der Schritte (c) bis (g) unter Ultraschall oder Mikrowellen durchgeführt wird.

3. Verfahren nach Anspruch 1 oder 2, wobei während der Schritte (c), (d) und (e) die Temperatur T_{d} 500°C +/- 10°C beträgt.

4. Verfahren nach einem der Ansprüche 1 bis 3, wobei während Schritt (d) die Temperatur T_{d} für eine Dauer D_{d} von weniger als 5 Minuten beibehalten wird.

5. Verfahren nach einem der Ansprüche 1 bis 4, wobei während der Schritte (e) und (f) die Temperatur T_{f} + 75°C +/- 10°C beträgt.

6. Verfahren nach einem der Ansprüche 1 bis 5, wobei während Schritt (f) das Funktionalisierungsmittel aus der Gruppe ausgewählt ist, die N-Methyl-N-tert-butyldimethyl-silyl]-trifluoracetamid (MTBSTFA), N,N-Dimethylformamid-dimethylacetal (DMF-DMA), Tetramethylammoniumhydroxid (TMAH), Trifluoressigsäureanhydrid (TFAA), Heptafluorbuttersäureanhydrid (HFBA), 2,2,2-Trifluorethanol (TFE), 2,2,3,3,4,4,4-Heptafluor-1-butanol (HFB), Methylchlorformat (MCF), Chlorameisensäureethylester (ECF) oder eine Mischung davon umfasst.

7. Verfahren nach einem der Ansprüche 1 bis 6, wobei während Schritt (f) das Lösungsmittel aus der Gruppe ausgewählt ist, die Wasser, lineare, verzweigte oder zyklische Alkane in C₁ bis C₈, R^{S}-OH Alkohole, wobei R^{S} ein Alkylradikal in C₁ bis C₈, ist, die Ketone R^{S1}-C (= O) -R^{S2}, die R^{S1}-O-R^{S2}-Ether, wobei R^{S1} und R^{S2} unabhängig voneinander Alkylradikale in C₁ bis C₈ sind, die gegebenenfalls einen Ring bilden, Isopropanol, Tetrahydrofuran (THF), Dioxan, Acetonitril, Dimethylformamid (DMF), Diethylformamid, Dimethylsulfoxid, Toluol, Essigsäure, Ameisensäure, Dichlormethan, Chloroform, Dichlorethan, Ethylacetat oder eine Mischung davon umfasst.

8. Verfahren nach einem der Ansprüche 1 bis 7, wobei während Schritt (f) die Temperatur T_{f} für eine Dauer D_{f} von 10 bis 30 Minuten beibehalten wird.

9. Verfahren nach einem der Ansprüche 1 bis 8, wobei während Schritt (g) die Temperatur Tᵥ in weniger als 30 Sekunden erreicht wird.

10. Verfahren nach einem der Ansprüche 1 bis 9, wobei in Schritt (g) die Temperatur Tᵥ 200°C + /- 20°C beträgt.

11. Verfahren nach einem der Ansprüche 1 bis 10, wobei während Schritt (g) die Temperatur Tᵥ für eine Dauer Dᵥ von 10 bis 30 Minuten beibehalten wird.

12. Verfahren nach einem der Ansprüche 1 bis 11, ferner umfassend, nach oder während des Schrittes (g), einen Übertragungsschritt (h) der verflüchtigten organischen Moleküle zu einem Analysegerät.

13. Verfahren nach Anspruch 12, wobei der Übertragungsschritt (h) der verflüchtigten organischen Moleküle durch Antrieb mittels eines Inertgases oder durch Absaugen bewirkt wird.

14. Verfahren nach Anspruch 12 oder 13, wobei die verflüchtigten organischen Moleküle an ein Gaschromatographie-Analysegerät übertragen werden.

15. Verfahren nach einem der Ansprüche 11 bis 14, wobei die verflüchtigten organischen Moleküle an das Gaschromatographie-Analysegerät (CPG), das an ein Massenspektrometer (MS) oder einen herkömmlichen Gaschromatographiedetektor gekoppelt ist, übertragen werden.

## Claims

1. Process for extraction, functionalization and volatilization of non-volatile organic molecules present in a solid medium, comprising the following steps:
(a) taking a solid soil sample;
(b) placing the sample under a pressure P_{d} of 0.1 to 200 kPa;
(c) raising the temperature of the sample, over the course of less than 30 seconds, from the temperature of the environment to a temperature T_{d} ranging from 250°C to 600°C;
(d) maintaining the sample at the temperature T_{d} for a period D_{d} of less than 15 minutes;
(e) decreasing the temperature of the sample, over the course of less than 30 seconds, from the temperature T_{d} to the temperature T_{f} ranging from 50°C to 100°C;
(f) adding to the sample at least one functionalizing agent and at least one solvent, and maintaining the sample at the temperature T_{f} for a period D_{f} of less than 1 hour; and
(g) heating the sample at a temperature Tᵥ ranging from 140°C to 300°C for a period Dᵥ of less than 1 hour, which causes volatilization of the functionalized organic molecules extracted from the soil sample.

2. Process according to Claim 1, in which at least one of steps (c) to (g) is carried out under ultrasound or under microwaves.

3. Process according to Claim 1 or 2, in which, during steps (c), (d) and (e), the temperature T_{d} is 500°C +/- 10°C.

4. Process according to any one of Claims 1 to 3, in which, during step (d), the temperature T_{d} is maintained for a period D_{d} of less than 5 minutes.

5. Process according to any one of Claims 1 to 4, in which, during steps (e) and (f), the temperature T_{f} is 75°C +/- 10°C.

6. Process according to any one of Claims 1 to 5, in which, during step (f), the functionalizing agent is chosen from the group comprising *N*-methyl-*N*-[tert-butyldimethylsilyl]trifluoroacetamide (MTBSTFA), *N*,*N*-dimethylformamide dimethylacetal (DMF-DMA), tetramethylammonium hydroxide (TMAH), trifluoroacetic anhydride (TFAA), heptafluoro-butyric anhydride (HFBA), 2,2,2-trifluoroethanol (TFE), 2,2,3,3,4,4,4-heptafluoro-1-butanol (HFB), methyl chloroformate (MCF), ethyl chloroformate (ECF) or a mixture thereof.

7. Process according to any one of Claims 1 to 6, in which, during step (f), the solvent is chosen from the group comprising water, linear, branched or cyclic C₁ to C₈ alkanes, R^{S}-OH alcohols wherein R^{S} is a C₁ to C₈ alkyl radical, R^{S1}-C (=O) -R^{S2} ketones, R^{S1}-O-R^{S2} ethers, wherein R^{S1} and R^{S2} are independently C₁ to C₈ alkyl radicals, optionally forming a ring, isopropanol, tetrahydrofuran (THF), dioxane, acetonitrile, dimethylformamide (DMF), diethylformamide, dimethyl sulphoxide, toluene, acetic acid, formic acid, dichloromethane, chloroform, dichloroethane, ethyl acetate, or a mixture thereof.

8. Process according to any one of Claims 1 to 7, in which, during step (f), the temperature T_{f} is maintained for a period D_{f} of 10 to 30 minutes.

9. Process according to any one of Claims 1 to 8, in which, during step (g), the temperature Tᵥ is reached in less than 30 seconds.

10. Process according to any one of Claims 1 to 9, in which, during step (g), the temperature Tᵥ is 200°C +/- 20°C.

11. Process according to any one of Claims 1 to 10, in which, during step (g), the temperature Tᵥ is maintained for a period Dᵥ of 10 to 30 minutes.

12. Process according to any one of Claims 1 to 11, also comprising, after or during step (g), a step (h) of transferring the volatilized organic molecules to an analysis instrument.

13. Process according to Claim 12, in which the step (h) of transferring the volatilized organic molecules is carried out by entrainment by means of an inert gas or by suction.

14. Process according to Claim 12 or 13, in which the volatilized organic molecules are transferred to a gas chromatography (GC) analysis instrument.

15. Process according to any one of Claims 11 to 14, in which the volatilized organic molecules are transferred to a gas chromatography (GC) analysis instrument coupled to a mass spectrometer (MS) or to a conventional (GC) detector.
